# EUROPEAN PATENT SPECIFICATION

(11) **EP 1 185 283 B1**
(45) Date of publication and mention of the grant of the patent: **07.09.2005**
(21) Application number: 00936234.4
(22) Date of filing: 24.05.2000
(51) Int. Cl.: A61K 38/00, A61K 38/16, A61P 37/06

(54) **NOVEL THERAPEUTIC USE OF VIRAL INFLAMMATION MODULATORY PROTEIN IN BLOCKING XENOGRAFT REJECTION**
NEUE THERAPEUTISCHE VERWENDUNG EINES EINE VIRALE ANSTECKUNG MODULIERENDES PROTEIN ZUR VERHINDERUNG VON ABSTOSSUNG VON FREMDTRANSPLANTATEN
NOUVELLE UTILISATION THERAPEUTHIQUE DE LA PROTEINE MODULATRICE DE L'INFLAMMATION VIRALE POUR BLOQUER LE REJET D'UNE XENOGREFFE

(30) Priority: 25.05.1999 US 136134 P
(43) Date of publication of application: 13.03.2002
(73) Proprietor: King Faisal Specialist Hospital and Research Centre, Riyadh 11211 (SA); UNIVERSITY OF LOUISVILLE RESEARCH FOUNDATION, INC., Louisville, KY 40292 (US)
(72) Inventor: KOTWAL, Girish, J., Louisville, KY 40241 (US); AL-MOHANNA, Futwan, 11211 Riyadh (SA); PARHAR, Ranjit, 11211 Riyadh (SA)
(74) Representative: Schüssler, Andrea
(86) International application number: PCT/US2000/014203
(87) International publication number: WO 2000/071147

(56) References cited:
- WO-A-98/13067
- US-A- 5 157 110
- US-A- 5 545 619
- US-A- 5 719 127
- KOTWAL G J ET AL: "IMP, a potential immunosuppressive and a future therapeutic in human diseases." EXPERIMENTAL AND CLINICAL IMMUNOGENETICS, vol. 14, no. 1, 1997, page 107 XP008016805 6th European Meeting on Complement in Human Disease;Innsbruck, Austria; March 12-15, 1997 ISSN: 0254-9670
- GARDNER C R: "THE PHARMACOLOGY OF IMMUNOSUPPRESSANT DRUGS IN SKIN TRANSPLANT REJECTION IN MICE AND IN OTHER RODENTS" GENERAL PHARMACOLOGY, PERGAMON PRESS, OXFORD, GB, vol. 26, no. 2, 1995, pages 245-271, XP002053689 ISSN: 0306-3623
- HOWARD J ET AL: "MOLECULAR MIMICRY OF THE INFLAMMATION MODULATORY PROTEINS (IMPS) OFPOXVIRUSES: EVASION OF THE IMFLAMMATORY RESPONSE TO PRESERVE VIRAL HABITAT" JOURNAL OF LEUKOCYTE BIOLOGY, FEDERATION OF AMERICAN SOCIETIES FOR EXPERIMENTAL, US, vol. 64, no. 1, 1998, pages 68-71, XP000909512 ISSN: 0741-5400
- MILLER C G ET AL: "THE COWPOX VIRUS-ENCODED HOMOLOG OF THE VACCINIA VIRUS COMPLEMENT CONTROL PROTEIN IS AN INFLAMMATION MODULATORY PROTEIN" VIROLOGY, ACADEMIC PRESS,ORLANDO, US, vol. 229, 1997, pages 126-133, XP002919492 ISSN: 0042-6822

## Description

### BACKGROUND OF THE INVENTION

### 1. Field of the Invention

The present invention relates to a method of blocking xenograft rejection in a patient in need of such treatment by administering to said patient a virally-encoded complement control protein, referred to as the inflammation modulatory protein (*IMP*). The present invention further relates to pharmaceutical compositions for the prevention or treatment of xenograft rejection comprising the IMP protein, alone or in combination with other immunosuppressive agents.The invention provides uses of the IMP protein, or a pharmaceutically acceptable salt thereof, for the manufacture of a medicament for preventing or treating xenograft rejection in a patient in need of such treatment.

### 2. Description of the Related Art

Transplantation of solid organs has enjoyed increasing success in the last two decades. This accomplishment has generated a problem: donor organ shortages have limited the number of patients that can be treated. Most accidents and illnesses causing death also damage the kidneys, heart, lungs, liver, and the pancreas, making them unsuitable for transplantation. The number of donors is further diminished by the reluctance of relatives to allow donation or by the discovery of AIDS or hepatitis in the potential donor. Thus, the number of patients waiting for organ transplantation far exceeds the number of available donor organs. Meanwhile, there has been little change in the supply of organ donors. Although this number has increased somewhat each year as the donor age limit was raised and awareness of organ donation increased, the plateau of heart and heart-lung transplantation reached after 1990 appears to be primarily related to limited donor availability. As a result, many patients die while awaiting a transplant, a number that continues to increase.

Transplantation of organs from nonhuman species (*xenografts*) would eliminate the shortage of cadaveric organs. When chimpanzee or baboon organs are transplanted into humans, rejection may be controlled successfully using immunosuppressive therapy currently used for human allografts: transplants between such closely related species are termed *concordant xenografts*. However, the widespread use of nonhuman primate organs for human transplantation is not practical. The necessary number of organs are not available because nonhuman primates have single births and a long period of gestation. There is also ethical opposition to the use of nonhuman primates.

Transplantation of organs from species distant to humans, such as swine, results in immediate fulminant hyperacute rejection within minutes to hours, in contrast to cell-mediated allograft rejection which takes place in seven to ten days. This is called *discordant transplant.* This extremely rapid rejection process is initiated by the deposition of pre-formed natural antibodies, primarily directed against the Gal carbohydrate epitope on the donor vascular endothelium, followed by activation of the host complement and coagulation cascades, leading to interstitial hemorrhage, intravascular coagulation, and ischemic necrosis. Such rejection cannot be satisfactorily controlled with currently available immunosuppressive drugs.

### The complement system.

The complement system involves approximately 30 plasma and membrane proteins that operate in a precise sequence to eliminate invading microorganisms. Complement activation can occur by any one of the three major pathways: Classical, Alternative, and Mannose-binding protein (*MBP*)*.* The classical pathway C1, C4, C2 and C3, is activated by antigen-IgG/IgM complexes as a result of the C1q portion of C1 binding to the Fc portion of IgG/IgM. The C1s portion of C1 causes a cleavage of C4 to a small C4a and large C4b molecule. C4b binds to the surface of microorganisms through covalent linkages and binds to C2a upon cleavage of C2s by C1s. The C4b2a complex is referred to as the C3 convertase of the classical pathway complex of enzymes that activates C3 by converting C3a to C3b. The C3b molecules covalently bind to surfaces of microorganisms forming clusters which can bind to complement receptor-bearing phagocytic cells.

The formation of C3b can also occur via the Alternative pathway. The Alternative pathway, consisting of factor B, D, H and I and properdin (P), is activated spontaneously by microorganisms in the absence of antibodies. There is mounting evidence that antibody enhances activation of the alternative pathway. The alternative pathway is initiated by the formation of a complex of factor B, a single-chain 93 kDa protein homologous to C2 with either C3b (formed during the classical pathway) or C3(H₂O) (formed when the internal thioester with bonds of circulating C3 undergo slow spontaneous hydrolysis). Factor B becomes susceptible to proteolysis by factor D (a 25 kDa serine protease). Factor D cleaves bound factor B, releasing a 33 kDa fragment (Ba) and leaving a 63 kDa fragment, Bb, attached to C3b or C3(H₂O). The resulting complex C3b or C3(H₂O)Bb is the alternative pathway convertase with the Bb fragment functioning as a serine protease capable of further cleaving C3 to C3b. The formation of C3b results in the activation of C5 by the action of C5 convertase to form C5a and C5b. The C5b molecule initiates the terminal pathway that culminates in the formation of the membrane attack complex (*MAC*). MAC forms large pores that resuit in cell lysis and can destroy certain types of microorganisms. The C5a causes the formation of chemoattractants and results in the influx of phagocytic cells and plasma proteins to the area of foreign substances that are activating complement. Thus, the complement system targets microorganisms or damaged host tissues resulting in an influx of phagocytic cells causing lysis.

The complement cascade can be blocked at multiple sites by purified bioactive vaccinia virus complement control protein (VCP), which is known to bind C3 and C4 (see Kotwal GJ, *et al*. (1997) Experimental and Clinical Immunogenetics, vol.14 (1) 107; Abstract F25, 6^{th} European Meeting on Complement in Human Disease; March 12-15 1997, Innsbruck, Austria).

Howard J *et al*. reported that cowpox virus (CPV) encodes several proteins that can influence the inflammatory response, and that models were being developed to test the efficacy of viral immunomodulators in alleviating inflammatory responses generated by mechanisms that included xenograft rejection (Howard J, *et al*. (1998) J Leukocyte Biology, vol. 64 (1) 68-71).

Complement control protein analogues of the important orthopoxviruses exhibit sequence similarities. Rodents are the natural reservoir hosts for cowpox virus (CPV): IMP is the homolog of VCP in CPV and significantly reduces the mouse model specific swelling response, preserving the tissue at the site of infection (viral habitat) (Howard J, *et al*. (1998) *supra*).

IMP modulates complement activity and it significantly diminishes the severity of the inflammatory response due to CPV in comparison to recombinant CPV lacking IMP (CPV-IMP), including the long term inflammatory response (Miller CG *et al*. (1997) Virology (Academic Press, Orlando USA) vol. 229 126-133). IMP has therapeutic potential for reducing inflammatory tissue damage in certain human conditions *e.g*. for preventing allograft rejection when used in combination with other immunosuppressive agents (Kotwal GJ, *et al*. (*supra*)).

### Hyperacute rejection and complement activation.

It is well known that humans have pre-formed antibodies, referred to as xenoreactive natural antibodies (XNA). These antibodies are thought to appear during the early neonatal period following coliform bacterial colonization of the large bowel. These antibodies react with the terminal Gal-alpha-1,3-Gal moiety and cross-react with porcine organs. These cross-reactive antibodies form an antigen-antibody complex, activating the classical complement pathway and causing hyperacute rejection. To overcome xenograft hyperacute rejection, several strategies have been developed. Among them, soluble decay accelerating factor (DAF) has been considered to be the most effective. DAF was injected into normal pigs, and transgenic pigs expressing human DAF have been produced. Transgenic human DAF seems to successfully inhibit complement-mediated damage to the endothelial cell, thus preventing endothelial activation and subsequent myocardial damage. This finding has led to the conclusion that because hyperacute rejection does not occur, human DAF makes a discordant species (pig) organ function as a concordant species organ. However, others have indicated that although both DAF and homologous restriction factor (*HRF20*) tend to prevent complement activation to some extent, its effectiveness is not sufficient for clinical use in transplantation. Studies have also shown that the alternative complement pathway can be activated despite the presence of membrane DAF and MCP membrane cofactor protein (*MCP*). Another problem with using natural human complement receptors like DAF and MCP is that these receptors also serve as proteins used by viruses and other microorganisms to gain entry into cells bearing these receptors. To effectively and safely inhibit complement and eliminate hyperacute rejection, other more potent complement inhibitory proteins are badly needed.

### SUMMARY OF THE INVENTION

Briefly, the present invention relates to a method for blocking xenograft rejection in a patient in need of such treatment comprising administering to said patient an effective amount of a protein of Formula (I):

signal sequence ↓→
M K E V S V T F L T L L G I G C V L S C C T I P S
R P I N M K F K N S V G T D A N A N Y N I G D T I E Y
L C L P G Y R K Q K M G P I Y A K C T G T G W T L F N
Q C I K R K C P S P R D I D N G Q I D I G G V E F G S
S I T Y S C N S G Y Q L I G E S K S Y C E L G Y T G S
M V W N P E A P I C E S V K C P S P P S V T N G R H N
G Y E D F Y T D G S V V T Y S C N S G Y S L I G N S G
I V C S G G E W S D P P T C Q I V K C P H P T I S N G
Y L S S G F K R S Y S H N D N V D F K C R H G Y K L S
G S S S S T C S P G N T W Q P E L P K C V R
[SEQ ID NO: 1].

The invention further relates to a pharmaceutical formulation for the prevention or treatment of xenograft rejection, which comprises a protein of the Formula (I) together with one or more pharmaceutical acceptable diluents, carriers or excipients therefor.

The present invention provides the use of a protein comprising the amino acid sequence of residues 20 to 263 of SEQ ID NO:1, or a pharmaceutically acceptable salt thereof, for the manufacture of a medicament for preventing or treating xenograft rejection in a patient in need of such treatment. The invention also provides such use where the amino acid sequence of said protein consists of the amino acid sequence of SEQ ID NO:1.

The invention further provides such uses, in which an immunosuppressive agent is coadministered to the patient, including but not limited to such use in which the medicament further comprises the immunosuppressive agent. The invention also provides such uses in which the immunosuppressive agent is selected from the group consisting of azathioprene, cyclosporine, and pharmaceutically acceptable salts thereof. A glucocorticoid may also be coadministered to the patient in such uses; the medicament may further comprise the glucocorticoid, which may be prednisone.

The invention further provides any of the foregoing uses, in which the medicament additionally comprises a pharmaceutically acceptable carrier, including but not limited to an aqueous carrier.

### BRIEF DESCRIPTION OF THE DRAWINGS

Figure 1. Elution of BSA (unbound fraction), lysozyme (2-2.5 M), and human heparin binds protein from a heparin (2.5-3 M) column (HITRAP heparin).
Figure 2. Elution of IMP from a HITRAP heparin column (102 M), 60 mg with concentrated medium from infected cells.
Figure 3. Further purification of heparin-eluted IMP using superdex 75 column showing pure bioactive IMP in fraction 4-6.
Figure 4. Uptake of fluorescein-labeled lysozyme by mast cells.
Figure 5. Uptake of fluorescein-labeled IMP by mast cells.
Figure 6. Uptake of fluorescein-labeled lysozyme by a mixture of mast cells and endothelial cells.
Figure 7. Uptake of fluorescein-labeled VCP by a mixture of mast cells and endothelial cells.
Figure 8. Analysis of complement inhibiting activity of fraction shown in Fig. 4. The VCP eluting at 1.0 M and 2.0 M are bioactive.
Figure 9. Mast/endothelial cell uptake of fluorescein-labeled VCP, lysozyme, MBP, IgG, and BSA. Phase-contrast images of mast/endothelial cells are accompanied by the fluorescent image of the same field for each labeled protein.
Figure 10. Inhibition of binding of anti-alpha-gal rhodamine conjugated antibody to pig aortal endothelial cells (PAECs) in the presence of alpha gal or IMP.
Figure 11. Flow cytometry showing inhibition of binding (decrease in fluorescence intensity) of anti-alpha gal antibody in the presence of IMP.
Figure 12. Evaluation of whether the binding of antibody to PAECs is specific only to the alpha gal residues on the surface or that the stearic hindrance does not discriminate specific from non-specific antibodies. Panel A: human endothelial cells treated with control antibody; Panel B: human endothelial cells treated with anti-human leukocyte antigen antibody conjugated with phycoerythrin; Panel C: human endothelial cells treated with IMP, PAECs, and anti-HLA I antibody.
Figure 13. Inhibition of killing of PAECs by IMP in the presence of serum alone, neutrophils alone, serum together with neutrophils, natural killer (NK) cells alone, NK + serum, or NK + serum + neutrophils.

### DETAILED DESCRIPTION OF THE PREFERRED EMBODIMENTS OF THE INVENTION

The present invention relates to a method of blocking xenograft rejection in a patient in need of such treatment by administering to said patient a virally-encoded complement control protein, referred to as the inflammation modulatory protein (*IMP*). This small protein is structurally related to the family of human complement control proteins that includes DAF and is functionally similar to the soluble complement receptor 1 (*sCR1*). Thus IMP can block complement at a very early stage by binding to C3b and C4b, by inhibiting the C3 convertase formation by either the classical or alternate pathway and also by accelerating the decay of the C3 convertase. In comparison to other complement inhibitory chemicals, IMP has several distinct advantages: 1) because of evolutionary pressure, it retains the most essential domains; 2) unlike sCR1 which cleaves C3 at two additional sites leaving cell bound fragments (possibly causing damage from immune cells with receptors for the fragments), IMP cleaves C3 at the first cleavage site in the presence of factor 1; and 3) the viral protein is small, and is not recognized as a foreign protein because it is structurally very similar to its homologs. The IMP protein for use in the present invention has the amino acid sequence of Formula (I):

signal sequence ↓
M K E V S V T F L T L L G I G C V L S C C T I P S
R P I N M K F K N S V G T D A N A N Y N I G D T I E Y
L C L P G Y R K Q K M G P I Y A K C T G T G W T L F N
Q C I K R K C P S P R D I D N G Q I D I G G V E F G S
S I T Y S C N S G Y Q L I G E S K S Y C E L G Y T G S
M V W N P E A P I C E S V K C P S P P S V T N G R H N
G Y E D F Y T D G S V V T Y S C N S G Y S L I G N S G
I V C S G G E W S D P P T C Q I V K C P H P T I S N G
Y L S S G F K R S Y S H N D N V D F K C R H G Y K L S
G S S S S T C S P G N T W Q P E L P K C V R
(SEQ ID NO: 1).

The amino acid abbreviations are set forth below:

| **Single-letter abbreviation** | **Three-letter abbreviation** | **Amino acid** |
|---|---|---|
| A | Ala | Alanine |
| C | Cys | Cysteine |
| D | Asp | Aspartic acid |
| E | Glu | Glutamic acid |
| F | Phe | Phenylalanine |
| G | Gly | Glycine |
| H | His | Histidine |
| I | Ile | Isoleucine |
| K | Lys | Lysine |
| L | Leu | Leucine |
| M | Met | Methionine |
| N | Asp | Asparagine |
| P | Pro | Proline |
| Q | Glu | Glutamine |
| R | Arg | Arginine |
| S | Ser | Serine |
| T | Thr | Threonine |
| W | Trp | Tryptophan |
| V | Val | Valine |
| Y | Tyr | Tyrosine |

One skilled in the art will recognize that certain amino acids are prone to rearrangement. For example, Asp may rearrange to aspartamide and isoasparagine as described in I. Schbn et al., *Int. J. Peptide Protein Res.* **14**: 485-94 (1979) and references cited therein. These rearrangement derivatives are included within the scope of the present invention. Unless otherwise indicated the amino acids are in the L configuration.

For purposes of the present invention, as disclosed and claimed herein, the following terms and abbreviations are defined as follows:
Base pair (bp) -- refers to DNA or RNA. The abbreviations A, C, G, and T correspond to the 5'-monophosphate forms of the nucleotides (deoxy)adenine, (deoxy)cytidine, (deoxy)guanine, and (deoxy)thymine, respectively, when they occur in DNA molecules. The abbreviations U, C, G, and T correspond to the 5'-monophosphate forms of the nucleosides uracil, cytidine, guanine, and thymine, respectively when they occur in RNA molecules. In double stranded DNA, base pair may refer to a partnership of A with T or C with G. In a DNA/RNA heteroduplex, base pair may refer to a partnership of T with U or C with G.
Chelating Peptide -- An amino acid sequence capable of complexing with a multivalent metal ion.
DNA -- Deoxyribonucleic acid.
EDTA -- an abbreviation for ethylenediamine tetraacetic acid.
ED50 -- an abbreviation for half-maximal value.
FAB-MS -- an abbreviation for fast atom bombardment mass spectrometry.
Immunoreactive Protein(s) -- a term used to collectively describe antibodies, fragments of antibodies capable of binding antigens of a similar nature as the parent antibody molecule from which they are derived, and single chain polypeptide binding molecules as described in PCT Application No. PCT/US 87/02208, International Publication No. WO 88/01649.
mRNA -- messenger RNA.
MWCO -- an abbreviation for molecular weight cut-off.
Patient -- a patient is any animal, usually a mammal, preferably a human.
Plasmid - an extrachromosomal self-replicating genetic element.
PMSF -- an abbreviation for phenylmethylsulfonyl fluoride.
Reading frame - the nucleotide sequence from which translation occurs "read" in triplets by the translational apparatus of tRNA, ribosomes and associated factors, each triplet corresponding to a particular amino acid. Because each triplet is distinct and of the same length, the coding sequence must be a multiple of three. A base pair insertion or deletion (termed a frameshift mutation) may result in two different proteins being coded for by the same DNA segment. To insure against this, the triplet codons corresponding to the desired polypeptide must be aligned in multiples of three from the initiation codon, i.e. the correct "reading frame" must be maintained. In the creation of fusion proteins containing a chelating peptide, the reading frame of the DNA sequence encoding the structural protein must be maintained in the DNA sequence encoding the chelating peptide.
Recombinant DNA Cloning Vector -- any autonomously replicating agent including, but not limited to, plasmids and phages, comprising a DNA molecule to which one or more additional DNA segments can or have been added.
Recombinant DNA Expression Vector -- any recombinant DNA cloning vector in which a promoter has been incorporated.
Replicon -- A DNA sequence that controls and allows for autonomous replication of a plasmid or other vector.
RNA -- ribonucleic acid.
RP-HPLC -- an abbreviation for reversed-phase high performance liquid chromatography.
Transcription -- the process whereby information contained in a nucleotide sequence of DNA is transferred to a complementary RNA sequence.
Translation -- the process whereby the genetic information of messenger RNA is used to specify and direct the synthesis of a polypeptide chain.
Tris -- an abbreviation for tris-(hydroxymethyl)aminomethane.
Treating -- describes the management and care of a patient for the purpose of combating the disease, condition, or disorder and includes the administration of a compound of present invention to prevent the onset of the symptoms or complications, alleviating the symptoms or complications, or eliminating the disease, condition, or disorder. Treating xenograft rejection therefore includes the inhibition of immune responses to the transplanted tissue.
Vector - a replicon used for the transformation of cells in gene manipulation bearing polynucleotide sequences corresponding to appropriate protein molecules which, when combined with appropriate control sequences, confer specific properties on the host cell to be transformed. Plasmids, viruses, and bacteriophage are suitable vectors, since they are replicons in their own right. Artificial vectors are constructed by cutting and joining DNA molecules from different sources using restriction enzymes and ligases. Vectors include Recombinant DNA cloning vectors and Recombinant DNA expression vectors.
X-gal -- an abbreviation for 5-bromo-4-chloro-3-idolyl beta-D-galactoside.

SEQ ID NO: 1 refers to the sequence set forth in the sequence listing and means a complement-inhibiting protein of Formula (I):

signal sequence ↓
M K E V S V T F L T L L G I G C V L S C C T I P S

R P I N M K F K N S V G T D A N A N Y N I G D T I E Y

L C L P G Y R K Q K M G P I Y A K C T G T G W T L F N

Q C I K R K C P S P R D I D N G Q I D I G G V E F G S
S I T Y S C N S G Y Q L I G E S K S Y C E L G Y T G S
M V W N P E A P I C E S V K C P S P P S V T N G R H N
G Y E D F Y T D G S V V T Y S C N S G Y S L I G N S G
I V C S G G E W S D P P T C Q I V K C P H P T I S N G
Y L S S G F K R S Y S H N D N V D F K C R H G Y K L S
G S S S S T C S P G N T W Q P E L P K C V R
(SEQ ID NO: 1).

The present invention provides a method for blocking xenograft rejection comprising administering to a patient in need of such treatment an effective amount of a compound of Formula (I) in a dose between about 1 and 1000 µg/kg. A preferred dose is from about 10 to 100 µg/kg of active compound. A typical daily dose for an adult human is from about 0.5 to 100 mg. In practicing this method, compounds of the Formula (I) can be administered in a single daily dose or in multiple doses per day. The treatment regime may require administration over extended periods of time. The amount per administered dose or the total amount administered will be determined by the physician and depend on such factors as the nature and severity of the disease, the age and general health of the patient and the tolerance of the patient to the compound.

The instant invention further provides pharmaceutical formulations comprising compounds of the Formula (I). The proteins, preferably in the form of a pharmaceutically acceptable salt, can be formulated for parenteral administration for the therapeutic or prophylactic treatment of xenograft rejection. For example, compounds of the Formula (I) can be admixed with conventional pharmaceutical carriers and excipients. The compositions comprising claimed proteins contain from about 0.1 to 90% by weight of the active protein, preferably in a soluble form, and more generally from about 10 to 30%. Furthermore, the present proteins may be administered alone or in combination with other anti-rejection agents or immunosuppresive agents, particularly those established as useful in preventing or treating allograft rejection. Preferred agents for use in combination with the protein of the present invention for preventing or treating xenograft rejection include azathioprine, glucocorticoids (such as prednisone), and cyclosporine.

For intravenous (IV) use, the protein is administered in commonly used intravenous fluid(s) and administered by infusion. Such fluids as, for example, physiological saline, Ringer's solution or 5% dextrose solution can be used.

For intramuscular preparations, a sterile formulation, preferably a suitable soluble salt form of a protein of the Formula (I), for example the hydrochloride salt, can be dissolved and administered in a pharmaceutical diluent such as pyrogen-free water (distilled), physiological saline or 5% glucose solution. A suitable insoluble form of the compound may be prepared and administered as a suspension in an aqueous base or a pharmaceutically acceptable oil base, *e.g*., an ester of a long chain fatty acid such as ethyl oleate.

The proteins for use in the presently claimed invention may be prepared by construction of the DNA encoding the claimed protein and thereafter expressing the DNA in recombinant cell culture. Techniques for making substitutional mutations at predetermined sites in DNA having a known sequence are we!! known, for example M13 primer mutagenesis. The mutations that might be made in the DNA encoding the protein of the present invention must not place the sequence out of reading frame and preferably will not create complementary regions that could produce secondary mRNA structure. See DeBoer et al., EP 75,444A (1983).

The protein of the present invention may be produced either by recombinant DNA technology or well known chemical procedures, such as solution or solid-phase peptide synthesis, or semi-synthesis in solution beginning with protein fragments coupled through conventional solution methods.

### A. Solid Phase

The synthesis of the protein of the present invention may proceed by solid phase peptide synthesis or by recombinant methods. The principles of solid phase chemical synthesis of polypeptides are well known in the art and may be found in general texts in the area such as Dugas, H. and Penney, C*., Bioorganic Chemistry*, Springer-Verlag, New York, pp. 54-92 (1981). For example, peptides may be synthesized by solid-phase methodology utilizing a PE-Applied Biosystems 430A peptide synthesizer (commercially available from Applied Biosystems, Foster City California) and synthesis cycles supplied by Applied Biosystems. Boc amino acids and other reagents are commercially available from PE-Applied Biosystems and other chemical supply houses. Sequential Boc chemistry using double couple protocols are applied to the starting p-methyl benzhydryl amine resins for the production of C-terminal carboxamides. For the production of C-terminal acids, the corresponding PAM resin is used. Arginine, Asparagine, Glutamine, Histidine and Methionine are coupled using preformed hydroxy benzotriazole esters. The following side chain protection may be used:

| | | | |
|---|---|---|---|
| Arg | Tosyl | Met | sulfoxide |
| Asp | cyclohexyl or benzyl | Ser | Benzyl |
| Cys | 4-methylbenzyl | Thr | Benzyl |
| Glu | cyclohexyl | Trp | formyl |
| His | benzyloxymethyl | Tyr | 4-bromo carbobenzoxy |
| Lys | 2-chlorobenzyloxycarbonyl | | |

Boc deprotection may be accomplished with trifluoroacetic acid (TFA) in methylene chloride. Formyl removal from Trp is accomplished by treatment of the peptidyl resin with 20% piperidine in dimethylformamide for 60 minutes at VC. Met(O) can be reduced by treatment of the peptidyl resin with TFA/dimethylsulfide/conHC1 (95/5/1) at 25°C for 60 minutes. Following the above pre-treatments, the peptides may be further deprotected and cleaved from the resin with anhydrous hydrogen fluoride containing a mixture of 10% m-cresol or m-cresol/10% p-thiocresol or m-cresol/p-thiocresol/dimethylsulfide. Cleavage of the side chain protecting group(s) and of the peptide from the resin is carried out at zero degrees Centigrade or below, preferably -20°C for thirty minutes followed by thirty minutes at 0°C. After removal of the HF, the peptide/resin is washed with ether. The peptide is extracted with glacial acetic acid and lyophilized. Purification is accomplished by reverse-phase C18 chromatography (Vydac) column in 0.1% TFA with a gradient of increasing acetonitrile concentration.

One skilled in the art recognizes that the solid phase synthesis could also be accomplished using the FMOC strategy and a TFA/scavenger cleavage mixture.

### B. Recombinant Synthesis

The protein of the present invention may also be produced by recombinant methods. Recombinant methods are preferred if a high yield is desired. The basic steps in the recombinant production of protein include:
a) construction of a synthetic or semi-synthetic (or isolation from natural sources) DNA encoding the protein of the present invention,
b) integrating the coding sequence into an expression vector in a manner suitable for the expression of the protein either alone or as a fusion protein,
c) transforming an appropriate eukaryotic or prokaryotic host cell with the expression vector, and
d) recovering and purifying the recombinantly produced protein.

### 2.a. Gene Construction

Synthetic genes, the *in vitro* or *in vivo* transcription and translation of which will result in the production of the protein may be constructed by techniques well known in the art. Owing to the natural degeneracy of the genetic code, the skilled artisan will recognize that a sizable yet definite number of DNA sequences may be constructed which encode the claimed proteins. In the preferred practice of the invention, synthesis is achieved by recombinant DNA technology.

Methodology of synthetic gene construction is well known in the art. For example, see Brown, *et al*. (1979) *Methods in Enzymology,* Academic Press, N.Y., Vol. 68, pp. 109-151. The DNA sequence corresponding to the synthetic claimed protein gene may be generated using conventional DNA synthesizing apparatus such as the Applied Biosystems Model 380A or 380B DNA synthesizers (commercially available from Applied Biosystems, Inc., 850 Lincoln Center Drive, Foster City, CA 94404).

It may desirable in some applications to modify the coding sequence of the claimed protein so as to incorporate a convenient protease sensitive cleavage site, e.g., between the signal peptide and the structural protein facilitating the controlled excision of the signal peptide from the fusion protein construct.

The gene encoding the claimed protein may also be created by using polymerase chain reaction (PCR). The template can be a cDNA library (commercially available from CLONETECH or STRATAGENE) or mRNA isolated from human adipose tissue. Such methodologies are well known in the art Maniatis, et al. *Molecular Cloning: A Laboratory Manual,* Cold Spring Harbor Press, Cold Spring Harbor Laboratory, Cold Spring Harbor, New York (1989).

### 2.b. Direct expression or Fusion protein

The claimed protein may be made either by direct expression or as fusion protein comprising the claimed protein followed by enzymatic or chemical cleavage. A variety of peptidases (e.g., trypsin) which cleave a polypeptide at specific sites or digest the peptides from the amino or carboxy termini (e.g., diaminopeptidase) of the peptide chain are known. Furthermore, particular chemicals (e.g., cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. See, *e.g*., Carter P., Site Specific Proteolysis of Fusion Proteins, Ch. 13 in *Protein Purification: From Molecular Mechanisms to Large Scale Processes,* American Chemical Soc., Washington, D.C. (1990).

### 2.c. Vector Construction

Construction of suitable vectors containing the desired coding and control sequences employ standard ligation techniques. Isolated plasmids or DNA fragments are cleaved, tailored, and religated in the form desired to form the plasmids required.

To effect the translation of the desired protein, one inserts the engineered synthetic DNA sequence in any of a plethora of appropriate recombinant DNA expression vectors through the use of appropriate restriction endonucleases. The claimed protein is a relatively large protein. A synthetic coding sequence is designed to possess restriction endonuclease cleavage sites at either end of the transcript to facilitate isolation from and integration into these expression and amplification and expression plasmids. The isolated cDNA coding sequence may be readily modified by the use of synthetic linkers to facilitate the incorporation of this sequence into the desired cloning vectors by techniques well known in the art. The particular endonucleases employed will be dictated by the restriction endonuclease cleavage pattern of the parent expression vector to be employed. The choice of restriction sites are chosen so as to properly orient the coding sequence with control sequences to achieve proper in-frame reading and expression of the claimed protein.

In general, plasmid vectors containing promoters and control sequences which are derived from species compatible with the host cell are used with these hosts. The vector ordinarily carries a replication site as well as marker sequences which are capable of providing phenotypic selection in transformed cells. For example, *E. coli* is typically transformed using pBR322, a plasmid derived from an *E. coli* species (Bolivar, *et al*., *Gene* **2**: 95 (1977)). Plasmid pBR322 contains genes for ampicillin and tetracycline resistance and thus provides easy means for identifying transformed cells. The pBR322 plasmid, or other microbial plasmid must also contain or be modified to contain promoters and other control elements commonly used in recombinant DNA technology.

The desired coding sequence is inserted into an expression vector in the proper orientation to be transcribed from a promoter and ribosome binding site, both of which should be functional in the host cell in which the protein is to be expressed. An example of such an expression vector is a plasmid described in Belagaje et al., U.S. patent No. 5,304,493, the teachings of which are herein are incorporated by reference. The gene encoding A-C-B proinsulin described in U.S. patent No. 5,304,493 can be removed from the plasmid pRB182 with restriction enzymes *Nde*l and *Bam*HI. The genes encoding the protein of the present invention can be inserted into the plasmid backbone on a *Nde*I/*Bam*HI restriction fragment cassette.

### 2.d. Procaryotic expression

In general, prokaryotes are used for cloning of DNA sequences in constructing the vectors useful in the invention.

For example, *E. coli* K12 strain 294 (ATCC No. 31446) is particularly useful. Other microbial strains which may be used include *E. coli* B and *E*. *coli* X1776 (ATCC No. 31537). These examples are illustrative rather than limiting.

Prokaryotes also are used for expression. The aforementioned strains, as well as *E. coli* W3110 (prototrophic, ATCC No. 27325), bacilli such as *Bacillus subtilis,* and other enterobacteriaceae such as *Salmonella typhimurium* or *Serratia marcescans,* and various *Pseudomonas* species may be used. Promoters suitable for use with prokaryotic hosts include the β-lactamase (vector pGX2907 [ATCC 39344] contains the replicon and β-lactamase gene) and lactose promoter systems (Chang *et al., Nature,* **275**:615 (1978); and Goeddel *et al., Nature* **281**:544 (1979)), alkaline phosphatase, the tryptophan (trp) promoter system (vector pATH1 [ATCC 37695] is designed to facilitate expression of an open reading frame as a trpE fusion protein under control of the trp promoter) and hybrid promoters such as the tac promoter (isolatable from plasmid pDR540 ATCC-37282). However, other functional bacterial promoters, whose nucleotide sequences are generally known, enable one of skill in the art to ligate them to DNA encoding the protein using linkers or adaptors to supply any required restriction sites. Promoters for use in bacterial systems also will contain a Shine-Dalgarno sequence operably linked to the DNA encoding protein.

### 2.e. Eucaryotic expression

The protein may be recombinantly produced in eukaryotic expression systems. Preferred promoters controlling transcription in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, *e.g.,* β-actin promoter. The early and late promoters of the SV40 virus are conveniently obtained as an SV40 restriction fragment which also contains the SV40 viral origin of replication. Fiers, *et al*., *Nature,* **273**:113 (1978). The entire SV40 genome may be obtained from plasmid pBRSV, ATCC 45019. The immediate early promoter of the human cytomegalovirus may be obtained from plasmid pCMBP (ATCC 77177). Of course, promoters from the host cell or related species also are useful herein.

Transcription of a DNA encoding the claimed protein by higher eukaryotes is increased by inserting an enhancer sequence into the vector. Enhancers are cis-acting elements of DNA, usually about 10-300 bp, that act on a promoter to increase its transcription. Enhancers are relatively-orientation and position independent having been found 5' (Laimins, L. *et* *al., PNAS* **78**:993 (1981)) and 3' (Lusky, M. L., *et. al., Mol. Cell Bio.* **3**:1108 (1983)) to the transcription unit, within an intron (Banerji, J. L. *et al., Cell* **33**:729 (1983)) as well as within the coding sequence itself (Osbome, T. F., *et al*., *Mol, Cell Bio.,* **4**:1293 (1984)). Many enhancer sequences are now known from mammalian genes (globin, RSV, SV40, EMC, elastase, albumin, α-fetoprotein and insulin). Typically, however, one will use an enhancer from an eukaryotic cell virus. Examples include the SV40 late enhancer, the cytomegalovirus early promoter enhancer, the polyoma enhancer on the late side of the replication origin, and adenovirus enhancers.

Expression vectors used in eukaryotic host cells (yeast, fungi, insect, plant, animal, human or nucleated cells from other multicellular organisms) will also contain sequences necessary for the termination of transcription which may affect mRNA expression. These regions are transcribed as polyadenylated segments in the untranslated portion of the mRNA encoding protein. The 3' untranslated regions also include transcription termination sites.

Expression vectors may contain a selection gene, also termed a selectable marker. Examples of suitable selectable markers for mammalian cells are dihydrofolate reductase (DHFR, which may be derived from the *Ba*/II/*Hind*III restriction fragment of pJOD-10 [ATCC 68815]), thymidine kinase (herpes simplex virus thymidine kinase is contained on the *Bam*HI fragment of vP-5 clone [ATCC 2028]) or neomycin (G418) resistance genes (obtainable from pNN414 yeast artificial chromosome vector [ATCC 37682]). When such selectable markers are successfully transferred into a mammalian host cell, the transfected mammalian host cell can survive if placed under selective pressure. There are two widely used distinct categories of selective regimes. The first category is based on a cell's metabolism and the use of a mutant cell line which lacks the ability to grow without a supplemented medium. Two examples are: CHO DHFR⁻ cells (ATCC CRL-9096) and mouse LTK⁻ cells (L-M(TK-) ATCC CCL-2.3). These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented medium. An alternative to supplementing the medium is to introduce an intact DHFR or TK gene into cells lacking the respective genes, thus altering their growth requirements. Individual cells which were not transformed with the DHFR or TK gene will not be capable of survival in nonsupplemented media.

The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, Southern P. and Berg, P., *J. Molec. Appl. Genet*. **1**: 327 (1982), mycophenolic acid, Mulligan, R. C. and Berg, P. *Science* **209**:1422 (1980), or hygromycin, Sugden, B. *et al., Mol. Cell. Biol*. **5**:410-413 (1985). The three examples given above employ bacterial genes under eukaryotic control to convey resistance to the appropriate drug G418 or neomycin (geneticin), xgpt (mycophenolic acid) or hygromycin, respectively.

A preferred vector for eucaryotic expression is pRc/CMV (commercially available from Invitrogen Corporation, 3985 Sorrento Valley Blvd., San Diego, CA 92121). To confirm correct sequences in plasmids constructed, the ligation mixtures are used to transform *E. coli* K12 strain DH5a (ATCC 31446) and successful transformants selected by antibiotic resistance where appropriate. Plasmids from the transformants are prepared, analyzed by restriction and/or sequence by the method of messing, *et al., Nucleic Acids Res.* **9**:309 (1981).

Host cells may be transformed with the expression vectors of this invention and cultured in conventional nutrient media modified as is appropriate for inducing promoters, selecting transformants or amplifying genes. The culture conditions, such as temperature, pH and the like, are those previously used with the host cell selected for expression, and will be apparent to the ordinarily skilled artisan. The techniques of transforming cells with the aforementioned vectors are well known in the art and may be found in such general references as Maniatis, *et al*., *Molecular Cloning: A Laboratory Manual,* Cold Spring Harbor Press, Cold Spring Harbor Laboratory, Cold Spring Harbor, New York (1989), or *Current Protocols in Molecular Biology* (1989) and supplements.

Preferred suitable host cells for expressing the vectors encoding the claimed proteins in higher eukaryotes include: African green monkey kidney line cell line transformed by SV40 (COS-7, ATCC CRL-1651); transformed human primary embryonal kidney cell line 293, (Graham, F. L. *et al., J. Gen Virol.* **36**:59-72 (1977), *Virology* **77**:319-329, *Virology* **86**:10-21); baby hamster kidney cells (BHK-21 (C-13), ATCC CCL-10, *Virology* **16**:147 (1962)); chinese hamster ovary cells CHO-DHFR- (ATCC CRL-9096), mouse Sertoli cells (TM4, ATCC CRL-1715, *Biol. Reprod.* **23**:243-250 (1980)); african green monkey kidney cells (VERO 76, ATCC CRL-1587); human cervical epitheloid carcinoma cells (HeLa, ATCC CCL-2); canine kidney cells (MDCK, ATCC CCL-34); buffalo rat liver cells (BRL 3A, ATCC CRL-1442); human diploid lung cells (WI-38, ATCC CCL-75); human hepatocellular carcinoma cells (Hep G2, ATCC HB-8065); and mouse mammary tumor cells (MMT 060562, ATCC CCL51).

### 2.f. Yeast expression

In addition to prokaryotes, eukaryotic microbes such as yeast cultures may also be used. *Saccharomyces cerevisiae*, or common baker's yeast is the most commonly used eukaryotic microorganism, although a number of other strains are commonly available. For expression in Saccharomyces, the plasmid YRp7, for example, (ATCC-40053, Stinchcomb, *et al., Nature* **282**:39 (1979); Kingsman *et. al., Gene* **7**:141 (1979); Tschemper *et al., Gene* **10**:157 (1980)) is commonly used. This plasmid already contains the trp gene which provides a selection marker for a mutant strain of yeast lacking the ability to grow in tryptophan, for example ATCC no. 44076 or PEP4-1 (Jones, *Genetics* **85**:12 (1977)).

Suitable promoting sequences for use with yeast hosts include the promoters for 3-phosphoglycerate kinase (found on plasmid pAP12BD ATCC 53231 and described in U.S. Patent No. 4,935,350, June 19, 1990) or other glycolytic enzymes such as enolase (found on plasmid pAC1 ATCC 39532), glyceraldehyde-3-phosphate dehydrogenase (derived from plasmid pHcGAPC1 ATCC 57090, 57091), zymomonas mobilis (United States Patent No. 5,000,000 issued March 19, 1991), hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein (contained on plasmid vector pCL28XhoLHBPV ATCC 39475, United States Patent No. 4,840,896), glyceraldehyde 3-phosphate dehydrogenase, and enzymes responsible for maltose and galactose (GALI found on plasmid pRY121 ATCC 37658) utilization. Suitable vectors and promoters for use in yeast expression are further described in R. Hitzeman *et al*., European Patent Publication No 73,657A. Yeast enhancers such as the UAS Gal from Saccharomyces cerevisiae (found in conjunction with the CYC1 promoter on plasmid YEpsec-hllbeta ATCC 67024), also are advantageously used with yeast promoters.

In a preferred embodiment, a DNA molecule encoding the protein of the present invention is as shown below. 
-51 TTTTTATTATTTGTACGATGTCCAGGATAACATTTTTACGGATAAATAAAT
ATGAAGGAGGTGAGCGTGACGTTCCTGACATTGTTGGGAATAGGATGCGTTCTATCATGCTGTACT 66
M K E V S V T F L T L L G I G C V L S C C T 22

ATTCCGTCACGACCCATTAATATGAAATTTAAGAATAGTGTGGTGACTGATGCTAATGCTAATTAC 132
I P S R P I N M K F K N S V G T D A N A N Y 44

AACATAGGAGACACTATAGAATATCTATGTCTACCTGGATACAGAAAGCAAAAAATGGGACCTATA 198
N I G D T I E Y L C L P G Y R K Q K M G P I 66

TATGCTAAATGTACAGGTACTGGATGGACACTCTTTAATCAATGTATTAAACGGAAATGCCCATCG 264
Y A K C T G T G W T L F N Q C I K R K C P S 88

CCTCGAGATATCGATAATGGCCAAATTGATATTGGTGGAGTAGAGTTTGGCTCTAGTATAACGTAC 330
P R D I D N G Q I D I G G V E F G S S I T Y 110

TCTTGTAATAGCGGATATCAATTGATCGGTGAATCTAAATCGTATTGTGAATTAGGATATACTGGA 396
S C N S G Y Q L I G E S K S Y C E L G Y T G 132

TCTATGGTATGGAATCCCGAGGCACCTATTTGTGAATCTGTTAAATGCCCATCCCCTCCATCTGTA 462
S M V W N P E A P I C E S V K C P S P P S V 154

ACCAACGGAAGACATAACGGATACGAGGATTTTTATACCGATGGGAGCGTTGTAACTTATAGTTGC 528
T N G R H N G Y E D F Y T D G S V V T Y S C 176

AATAGTGGATATTCGTTGATTGGTAACTCTGGTATCGTGTGTTCAGGAGGAGAATGGTCCGATCCA 594
N S G Y S L I G N S G I V C S G G E W S D P 198

CCCACGTGTCAGATTGTTAAATGTCCACATCCTACAATATCAAACGGATACTTGTCTAGCGGGTTT 660
P T C Q I V K C P H P T I S N G Y L S S G F 220

AAAAGATCATACTCACACAACGACAATGTAGACTTTAAGTGCAGGCACGGATATAAACTATCTGGT 726
K R S Y S H N D N V D F K C R H G Y K L S G 242

TCCTCATCATCTACTTGCTCTCCAGGAAATACATGGCAGCCGGAACTTCCAAAATGTGTACGC 792
S S S S T C S P G N T W Q P E L P K C V R 264
 (SEQ ID NO: 2). This DNA molecule is homologous to the sequence disclosed in U.S. Patent 5,157,110, the contents of which are incorporated herein by reference.

The following examples are presented in order to more fully illustrate the preferred embodiments of the invention. They should in no way be construed, however, as limiting the broad scope of the invention.

### EXAMPLE 1: Cloning of DNA encoding IMP in a yeast expression vector.

The goal of this project is to achieve expression of large quantities of authentically folded, stable and functional IMP using the Pichia yeast expression system (Invitrogen), and to purify the protein to homogeneity. The rationale for choosing the Pichia expression system over other eucaryotic expression systems is the high expression levels, simplicity, ease of cloning and culture, eucaryotic co-translational modification (IMP is non-glycosylated but requires cleavage of the signal sequence and secretion which can be achieved easily by this system), and authentic folding (IMP has at least 6 disulfide bonds, and a bacterial system is not ideal for the folding of such as a protein).

The DNA encoding the IMP protein was amplified using primers derived from the ends of the open reading frame from genomic DNA for cowpox virus and was digested with the restriction, enzyme Hinc II. The fragment was then purified from low melting point agarose gel as described earlier and the ends were mutagenized by PCR using modified oligonucleotide primers to insert restriction enzymes. This modified DNA of IMP, lacking the region encoding the signal sequence with the appropriate restriction enzymes, was inserted into the multiple cloning sites of the expression vector pPIC9, which fuses a signal sequence to the insert ORF. In addition, this vector carries an ampicillin resistance gene, ColE1, and F1 origins of replication.

The recombinant plasmid was then selected, characterized by restriction analysis, amplified in *E. coli* to transform *Pichia pastoris,* screened for integration, and sequenced. The insert, carried by the recombinant plasmid with the correct sequence was then purified. The vector carrying the 5' and 3' AOX1 (Alcohol dehydrogenase) sequences which target integration into the Pichia host genome resulted in exchange of the native AOX1 gene with the IMP gene. To select for integrants, the HIS4 deficient strain, was used in the presence of histidine free medium. Cells in which recombination occurred grew, while cells not producing histidine did not survive. In order to screen for integration at the correct loci, colonies from the HIS-free plate were spotted onto a minus HIS plate, plus glycerol plate and a minus HIS, plus methanol plate. Colonies exhibiting sluggish growth on methanol no longer have the AOX1 gene and have a HIS+, methanol- phenotype. The selected colonies were then grown for 2 days in media containing glycerol as the sole source of carbon. The cells were harvested 2 days later and used to induce expression. The harvested cells were resuspended in media containing methanol. At several time points, post-induction samples of clarified culture media were prepared for analysis. This was done on a 10-20% gradient gel and stained by a silver staining procedure with known amounts of gamma globulin run side by side with expressed VCP. The results obtained confirm that protein of the correct size is secreted and has been properly purified for use.

### EXAMPLE 2: Isozyme-like heparin binding activity of VCP

VCP has isozyme-like heparin-binding activity that allows uptake by mast cells, which could then result in a sustained release at the site of infection. An analysis of the sequence of VCP suggests a possible molecular basis for the heparin binding activity. This includes an overall positive charge of the protein (Pi of 8.8) and the presence of putative heparin binding sites (Lys X Lys) (where X is only amino acid).

Medium from RK-13 cells infected with vaccinia virus WR strain was harvested, concentrated and passed over DEAE biogel column. The fractions from DEAE containing VCP were pooled and purified on a HITRAP heparin column. The bound proteins were washed with phosphate buffered saline and eluted with increased sodium chloride concentrations ranging from 100 mM to 2.0 M. The fractions were then separated on a gradient polyacrylamide gel and then silver stained. Purified BSA (69 kDa), lysozyme (14.4 kDa), and heparin binding protein (*HBP*)(36 kDa) were mixed in PBS and similarly separated on a heparin column to determine the salt concentrations at which they elute, to determine the approximate heparin-binding activity of VCP. A hemolysis assay to determine bioactivity of VCP containing fractions was performed.

One hundred µg of purified VCP and lysozyme were dissolved in 10 µl of 50 ml bicarbonate buffer, pH 8.5. It was then mixed with 0.5-1.0 µl of fluorescein in DMSO and incubated on ice for 2 hours. At the end of the incubation period 2 ml of PBS was added and the solution was concentrated to 100 µl on an Amicon microconcentrator and kept at 4°C until used.

Human mast cells (HMC-1) were cultured in RPMI medium containing 10%serum. HMC-1 were mixed with 20 µl of either fluorescein-labeled VCP or lysozyme. After 1 hour incubation at room temperature, cells were washed and suspended in 10 µl of glycerated saline, placed on a slide, and a cover slip was placed on the drop and observed under UV light microscope.

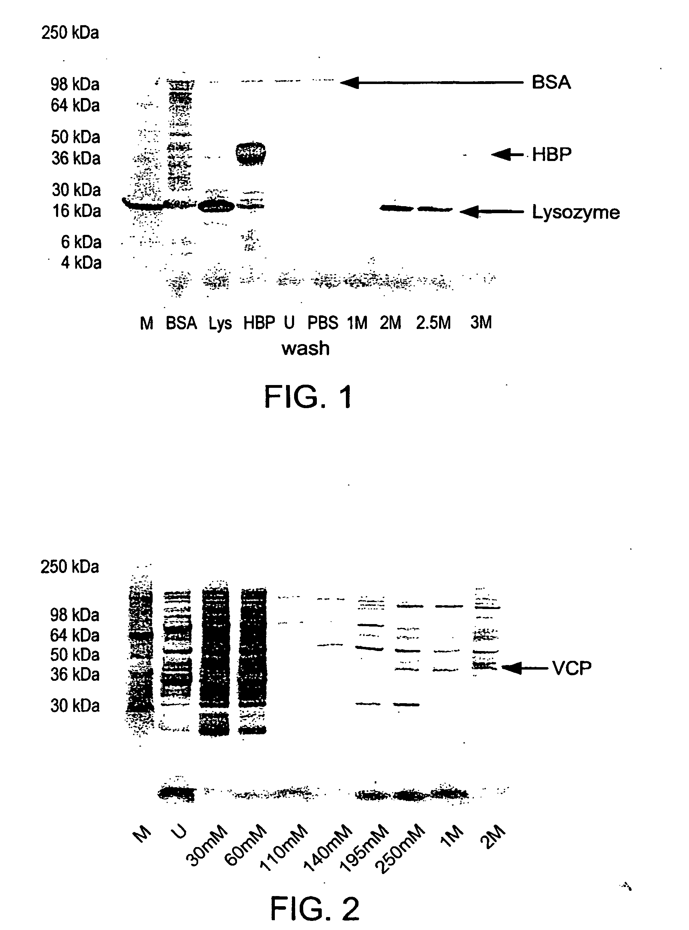

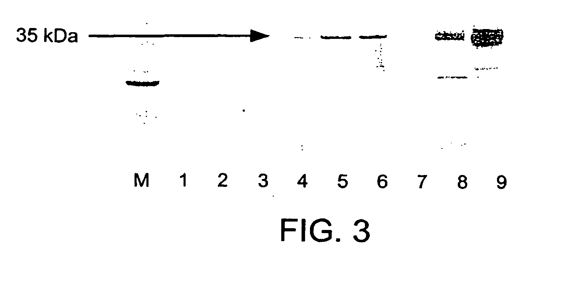

The results of the analysis of the fractions eluting from the two separate heparin columns, shown in Figs. 1-3, suggesting that the BAS does not bind and therefore elutes with the wash. In the top part of Figs. 1-3, 1.0 M refers to the molecular weight marker. BSA contains our purified BSA, Lys contains purified lysozyme. HBP lane contains pure HBP. U is the unbound fraction, and the rest of the lanes are washes washed with increasing NaCl. Lysozyme elutes at 2.0 M and 2.5 M. VCP, on the other hand (the bottom gel), eluted at 2.0 M, while the heparin binding protein eluted at about 3.0 M. The VCP eluting at 1.0 and 2.0 M inhibited 80% of the hemolysis of sensitized sheep red cells in the presence of human serum, indicating that fractions containing the visible VCP bands were bioactive. We conclude from this data that VCP has lysozyme-like heparin binding activity,

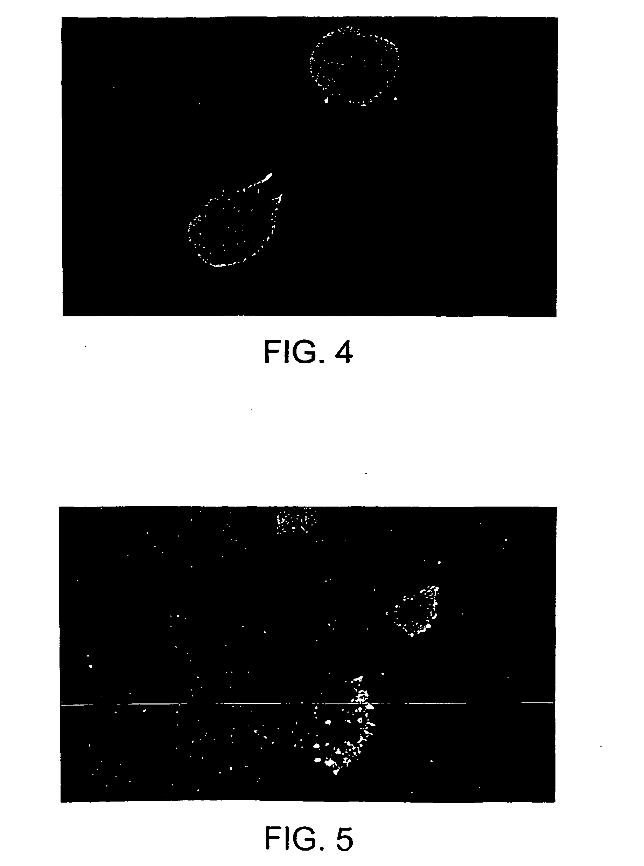

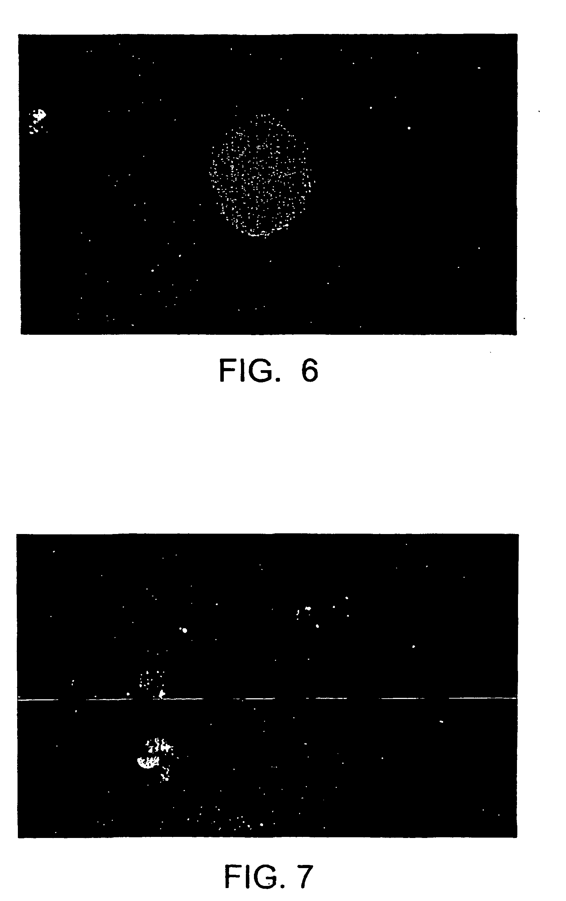

To determine whether VCP had lysozyme-like ability to be taken up by mast cell granules, fluorescein-labeled VCP and lysozyme were mixed with HMC-1. As can be seen from Figs. 4-7, both lysozyme and VCP were taken up, and the cells were fluorescent. BSA, used as negative control, had no fluorescence. These data suggest that VCP at the site of infection could be taken up by the mast cell granules and released over a long period so as to have sustained inhibition of complement activity in the tissue.

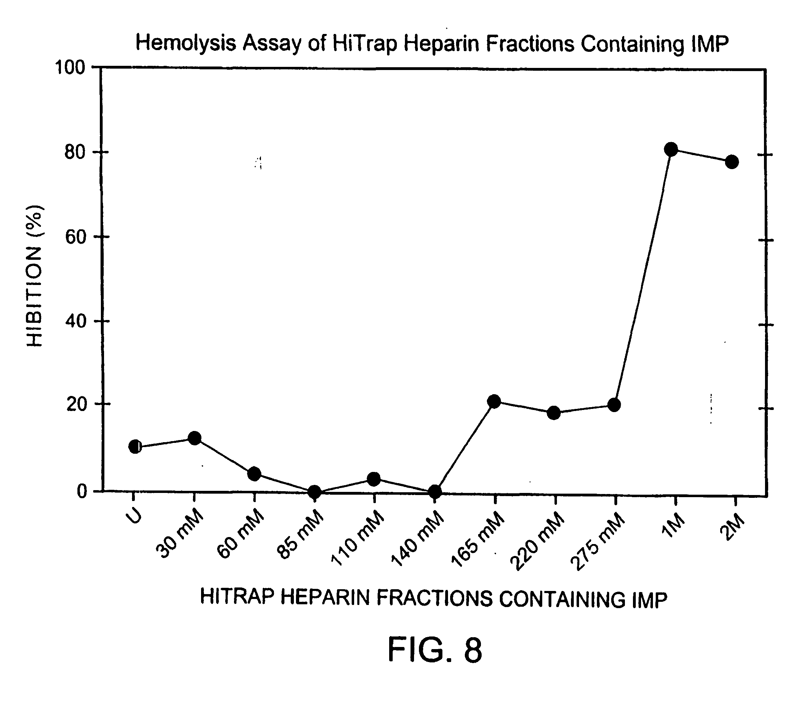

To further determine the ability of VCP, analysis of complement inhibition activity of fraction shown in Fig. 1 has revealed that the activity is mainly observed in fraction 1 M and 2M (Fig. 8).

To determine the molecular basis of the heparin binding activity of the proteins, we analyzed the putative binding sites for lysozyme, VCP and heparin binding protein. We conduded that the heparin binding can be attributed to the overall basic property of these proteins. The presence of basic amino acids in dose proximity at several sites within the proteins may also contribute lo heparin binding. When VCP and HBP were compared there seems to be a LysXLys site in each protein (X is any amino acid).

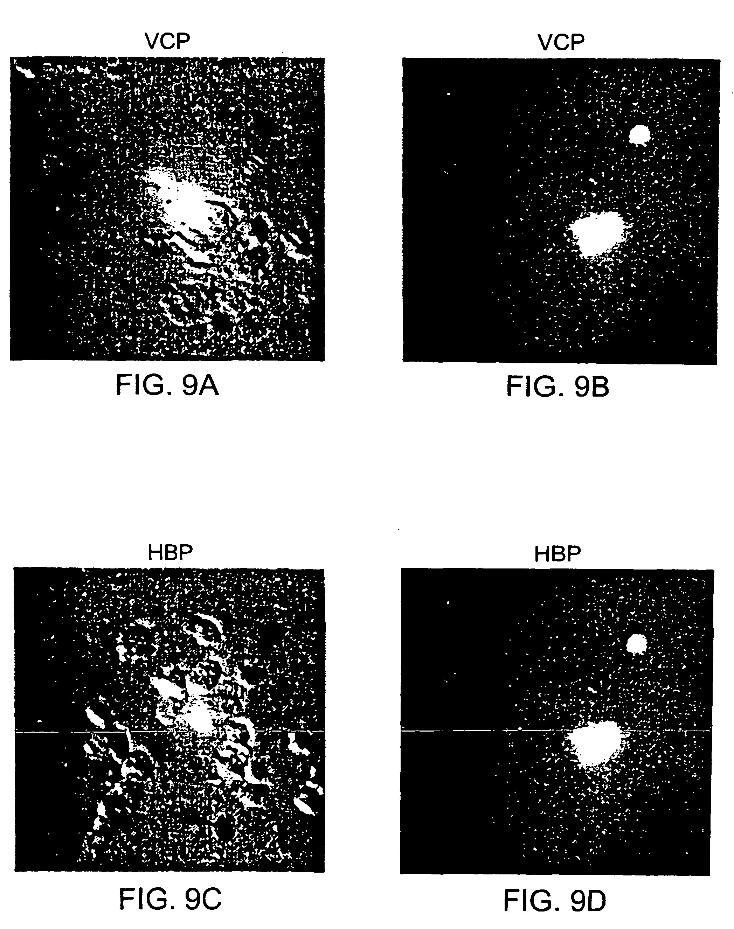

To further determine the ability of VCP to be taken up by mast cells and endothelial cells in a mixed culture, fluorescein labeled VCP, lysozyme, HBP, IgG, and BSA were mixed with HMC-1 and HUVEC. Lysozyme and HBP were included as positive controls due to their ability to bind heparin and be taken up by mast cells. BSA and IgG were included as negative controls because of their weak heparin binding properties. Fig. 9 shows the phase-contrast image and the corresponding fluorescent image for each of the protein/cell mixtures. VCP was taken up by the mast/endothelial cells and exhibited a fluorescent intensity similar to that of lysozyme and HBP. Conversely, the fluorescent intensities of IgG and BSA were significantly lower, suggesting they did not bind heparin and were not taken up by the mast/endothelial calls as efficiently as VCP, lysozyme, or HBP. These data suggest that VCP at the site of infection could be taken up by mast cell granules, which could subsequently be taken up by endothelial cells, and released over time resulting in sustained inhibition of complement activity in the tissue.

Chemokines are small chemoattractant cytokines which are critical for the recruitment of leukocytes to sites of inflammation. Chemokines interact via their amino terminus with receptors located on leukocytes, while their carboxy terminus binds to glycosamines, such as heparin, located on endothelial cell surfaces.

Through heparin binding, chemokines are able to establish gradients along endothelial walls and direct leukocyte localization and migration into tissues. Therefore, the inventors tested the ability of VCP to bind to heparin on endothelial cells and inhibit migration of monocytes in the presence and absence of the chemokine MIP- 1α. Table 1 shows the number of endothelial cells with monocytes attached under each of the conditions tested.

**Table 1.**

| Percent reduction of monocyte attachment to endothelial cells in the presence of VCP | | |
|---|---|---|
| | **# MONOCYTES ATTACHED** | **% DECREASE** |
| No MIP-1α or VCP | 0 | -- |
| 1×MIP-1α | 12 | -- |
| 1×MIP-1α + 1×VCP | 7.3 | 39.2 |
| 1×MIP-1α + 2×VCP | 4.7 | 60.8 |
| 2×MIP-1α | 24.7 | -- |
| 2×MIP-1α + 2×VCP | 3 | 87.9 |
| 4×MIP-1α | 10.3 | -- |
| 4×MIP-1α + 4×VCP | 5 | 51.5 |
| 4×MIP-1α + 8×VCP | 7 | 32 |
| 4×MIP-1α + 16×VCP | 2 | 80.6 |

Each number under the "# Monocytes Attached" column in Table 1 represents an average of 3 samples, except for the 4×, 8× and 16× VCP samples which were performed only once. The "% Decrease" column in Table 1 refers to the decrease in relation to the MIP-1α only control for each respective sample group. Inclusion of VCP resulted in significantly lower levels of monocyte migration, with the most dramatic decrease (87.9%) occurring when 2× VCP was included with 2× MIP-1α. VCP appears to bind to heparin on endothelial cells, blocking the attachment of chemokines, and therefore, preventing leukocyte localization.

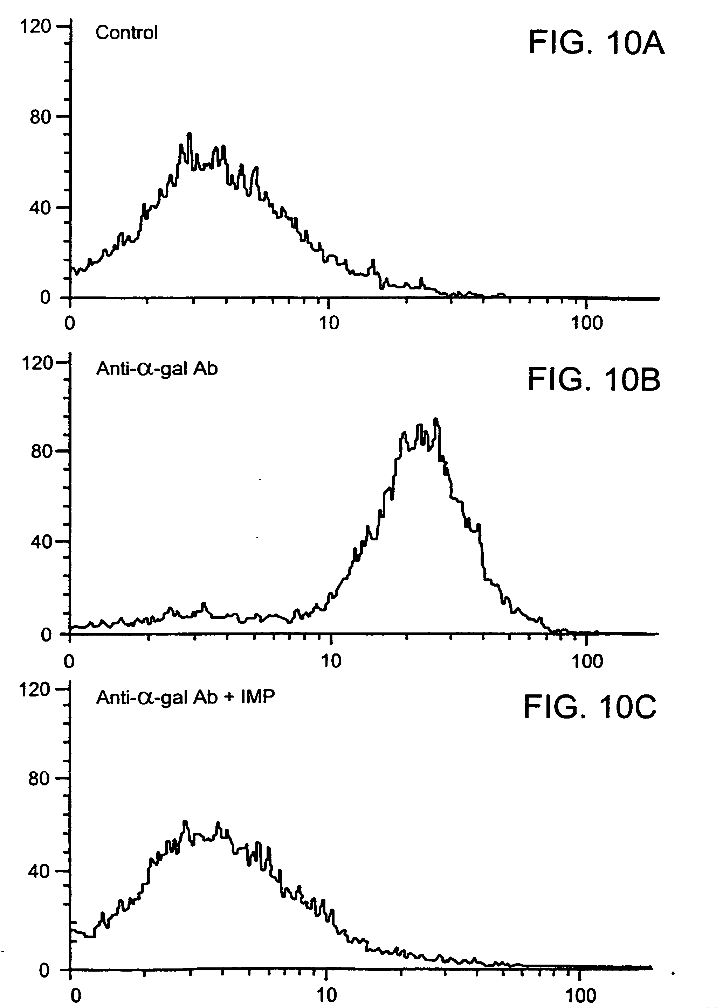

In order to determine whether IMP can block the complement-mediated killing of pig aorta endothelial cell suspension in the presence of human serum, a lyophilized preparation containing VCP from a natural infection was able to block the human serum-induced and NK cell-induced killing of pig aorta endothelial cells. This clearly proves the potential of IMP in preserving xenotransplants (Fig. 10). VCP and IMP are functionally identical and therefore practically interchangeable.

### EXAMPLE 3: Overexpression and purification of large quantities of IMP in a suitable system.

This study will use the yeast Pichia system to provide an authentic product in large quantities and regulate inflammation in a viral-induced inflammation-model. The standard methodology for purification and analysis of complement-inhibitory activity of VCP from a natural infection is described briefly as follows:

RK-13 cells (ATCC CCL 37, American Type Culture Collection, Rockville, MD) are grown to confluency in 40 150-cm³ cell culture flasks with Eagle's minimum essential medium (MEM) containing 10% fetal bovine serum (FBS) in a CO₂ incubator maintained at 37°C. The cells are infected with vaccinia virus strain WR (ATCC VR-119) in 2.5 ml of MEM containing 2% FBS for 2 hr. at a multiplicity of infection of 30 pfu (plaque forming units)/cell. The cell monolayer is washed extensively with serum-free medium in order to remove the inoculum and residual serum proteins. The washed cells are placed into a flask containing 10 ml of serum-free MEM or opti-MEM, then incubated at 37°C for 24 hr. The medium is harvested and clarified by low-speed centrifugation (2500 rpm in an H6000A rotor (DuPont Sorvall, Newtown, CT) in a Sorvall RC-3B centrifuge (DuPont Sorvall)) for 10 minutes at 4°C. The pooled medium is then concentrated tenfold using a 500-ml stirred cell under N₂ pressure (75 psi) with a washed YM10 membrane (Amicon, Beverly, MA). The medium is further concentrated in a 50-ml stirred cell (Amicon), using a washed YM10 membrane. The concentrate is diluted to 50 ml with a buffer containing 30 mM NaC!, 10 mM EDTA, 10 mM Tris-HCl, pH 8.6, and reconcentrated. This step helps in desalting the proteins and equilibrates them in the column binding buffer. The concentrate is applied to a DEAE Biogel column (Bio-Rad Laboratories, Diagnostic Group, Hercules, CA), equilibrated with the above buffer, then eluted with a step gradient of 0.03 to 0.3 M NaCl. Two-milliliter fractions are placed individually into Centricon-10 concentrators (Amicon), concentrated tenfold, and stored at -90°C. Fractions are monitored by SDS PAGE. Those containing VCP are applied to a Sephadex G-100 column (Pharmacia Biotech, Inc., Piscataway, NJ), equilibrated with 141 mM NaCl, 0.15 mM CaCl₂, 0.5 mM MgCl₂, 1.8 mM sodium barbital, and 3.1 mM barbituric acid, pH 7.3 to 7.4.

The fractions are again analyzed by SDS PAGE, either by staining with silver stain and by Western transfer or by mixing radiolabeled proteins with unlabeled proteins and monitoring the radioactivity by exposure of the dry gel to X-ray film. The complement inhibitory activity of the protein is measured by standard procedures and the following commercially available materials: sensitized sheep red blood cells (Diamedix, Miami, FL), in 150-µl volumes dispensed into the wells of a 96- Microwell plate (Nunc, Inc., Naperville, IL). A blank is prepared by adding 50 µl of diluent (gelatin veronal buffer, Sigma, St. Louis, MO) to the first well. A reference standard is prepared by adding 5 µl of a 1:20 diluted standard serum (Diamedix) and 45 µl of diluent. Samples (15 µl) of purified material, 5 µl of 1:20 diluted standard serum, and 30 µl of diluent are added to the test well. The contents of the well are mixed, and the plate is incubated at 37°C for 1 hr. The samples are withdrawn from the wells and centrifuged in microfuge tubes, The supernatants are transferred to a flat-bottom microplate. Adsorbance is measured at 415 nm. The percent inhibition of hemolysis is calculated by deducting the percent hemolysis from 100.

The recombinant yeast *Pichia pastoris* vector expressing IMP has already been isolated, and the IMP will be purified from the supernatant by a procedure identical to the purification method used for the natural infection system.

### EXAMPLE 4: The effect of IMP on complement inhibition.

This study is designed to confirm *in vivo* the results of *in vitro* experiments indicating that IMP effectively inhibits complement activation.

### 1. Experimental design.

A) Inhibition of inflammatory response. *In vivo* injection of IMP into mouse connective tissue air pouches containing recombinant CPV-IMP in BALB/c, C5-deficient and matched C5-sufficient/C3 -deficient and C3-sufficient mice. The connective and surrounding tissue will be examined for specific cellular changes.
   Two sensitive *in vivo* assays have been developed to measure the extent and nature of the inflammatory response. One of the two is a footpad injection method, which involves injecting 10⁶ virus particles of the recombinant and wild type viruses in the right footpad of individual mice and then monitoring the SSR for up to two weeks. Generally the peak response is seen around the 10th day and there is about 200% greater SSR in the mutant lacking in the IMP as there is with the wild type virus injection. At least 24 BALB/c mice per each of the four groups (96 mice) will be required to obtain statistically significant results. The 24 mice will be used as follows: six for footpad measurement; six for air pouch experiments to quantitate the influx of cells subsequent to hematoxylin and eosin staining; four for measurement of myeloperoxidase (indication of neutrophil influx) directly from connective tissue, with a sensitive in-house developed microplate assay; and four each will be used for measurement of CD4+ or CD8+ cells by immunohistochemistry. Previous experience has shown the SD of the footpad SSR to be very low (~0.25 mm) and the model highly reproducible. In the other method, age-matched mice will be injected subcutaneously on the dorsum with 1 cc of air, using a 27-gauge needle. This will be followed by injection of 10⁶ pfu of gradient purified CPV or CPV-IMP together with and without appropriate amounts of IMP or PBS in a 100 µl volume of PBS, directly into the air pouch using a 25-gauge needle. After 10 days, the mice will be sacrificed by exsanguination. Air pouch connective tissue will then be quickly removed and placed on a slide, fixed with 99% methanol, then stained with May-Grunwald-Giemsa for differentiation and quantitation. Tissue adjacent to the CT pouch will also be taken and then longitudinal sections will be prepared and stained with hematoxylin and eosin to examine the extent and type of tissue infiltration. These *in vivo* procedures will allow quantitation by enumerating inflammatory cells/cm².
B) C3-binding assay. Mouse splenocytes (3 × 10⁵, prepared according to the technique described in the next study) are incubated in the presence of pooled normal human serum (NHS; 0-12.5%) in MT-PBS for 10 minutes at 37°C. Reactions are stopped by the addition of EDTA to 10 mM and cells are collected by centrifugation (250 × g, 5 minutes, 4°C). To detect C3 deposition, cells are incubated (30 minutes on ice) with FITC-conjugated rabbit anti-human C3c Fab fragments (Boehringer) at 1:50 dilution in MT-PBS containing 2% HI-FCS and 10% mouse serum and analyzed by flow cytometry,
   In the study group, IMP will be added into incubation solution. The control group include spienocytes incubated with anti-C3c antibody (no serum) to control for nonspecific antibody deposition. Quantitation of C3 deposition is performed by flow cytometry; results are expressed as mean channel fluorescence (MCF) of triplicate samples.
C) Animal groups studied. For inhibition of inflammatory response, four groups of mice will be studied, and each group will include 24 mice.
   Group 1. C5-deficient mice
   Group 2. Matched C5-suffcient mice
   Group 3. C3-deficient mice
   Group 4. Matched C3-sufficient mice,

### 2. Results.

It is expected that IMP will cause much less severe complement activation and less tissue infiltration. In splenocyte study, less complement deposition will be anticipated, This study will establish the basis for mouse-to-rat heart xenotransplantation.

### EXAMPLE 5. Mouse-to-rat xenogeneic heart transplantation.

### 1. Rationale for the study

This study is designed to test the activity of complement during mouse-to-rat heart xenotransplantation. Ordinary mouse-to-rat transplantation (concordant) is used to compare with mouse-to-rat xenotransplantation in which the recipient rats have been sensitized by splenocyte injection.

### 2. Sensitization of the rats using splenocytes from mice.

A. Preparation of donor spleen cells. The mice are anesthetized with sodium pentobarbital (50-55 mg/kg, ip). The abdomen is opened and the spleen is removed through a midline incision under aseptic condition. The spleen is transferred to a sterile petri dish containing 5 ml RPMI 1640 medium (Sigma Chemical Co.) and minced by a tissue homogenizer and suspended in RPMI medium. The suspension is passed through a sterile filter to eliminate stromal elements. The filtrate is then centrifuged at 1100 rpm for 10 minutes, the cell button is resuspended in RPMI, and the splenocyte suspension is applied to a Ficoll-Hypaque gradient and centrifuged at 1600 rpm for 15 minutes. Red blood cells are lysed with NH₄Cl. Any remaining white blood cells are washed with RPMI 1640 medium. Viability of the cells will be evaluated by trypan blue dye exclusion. If viability is greater than 90%, the cells are used for injection.
B) Pretreatment of graft recipients. The recipient rats will be injected with 25×10⁶ mouse splenocytes each in a volume of 0.01 ml of RPMI 1640 medium. This volume will be injected percutaneously into the thymus with the needle angled under the manubrium.

### 3. General surgical procedure

A) Time of heart transplantation: 4 weeks after receiving mouse splenocyte injection.
B) Instruments and preoperative preparations.
   Operating microscope: Carl-Zeiss Surgical Microscope
   Sutures: II-0 Prolene with a 3-mm curved round-bodied -needle.
C) Both donor and recipient operations are performed under sterile conditions.
D) Penicillin (10,000 units) is administered i.m. at the time of surgery.

### 4. Donor operation.

Anesthesia is induced with sodium pentobarbital (55 mg/kg, i.p.). Twenty units of heparin is injected via the penile vein. The chest is opened through a median stemotomy. Under the microscope (16×), the right superior vena cava and the inferior vena cava are ligated with 6-0 silk suture near the atrium and divided distally to the ligatures. The ascending aorta and a segment of the innominate artery are immobilized. The aorta is ligated and cut close to the innominate artery, and a 1 mm length of the innominate artery is left attached to the aorta for late revascularization. The main pulmonary artery is freed and transacted at the point of bifurcation. The left superior vena cava and pulmonary veins are ligated en masse and divided distally. The left atrium is ligated to reduce its size. The heart is then excised, perfused by 4°C cardioplegia (Euro-Collins or St. Thomas solution), and immersed in the cardioplegic solution (2 - 4°C) for transplantation.

### 5. Cervical heterotropic translation.

The recipient rat is anesthetized with sodium pentobarbital (55 mg/kg i.p.) and placed in a supine position, with the head facing the operator. The head is stretched and immobilized with a piece of rubber band anchoring the upper incisor teeth of the animal to the operating board. After shaving, a vertical incision, about 1.5 cm long, is made in the neck from the midpoint of the chin to the right mid-clavicle. Under the microscope (16×) the external jugular vein is mobilized from the clavicle to its first major bifurcation. All the small branches of this segment are cut with the fine-tip cautery. The jugular vein is proximally clamped with the fine clamp close to the clavicle and then transacted distally at the point of bifurcation. The stemomastoid muscle is transected to expose the right common carotid artery. The vessel is mobilized and clamped proximally with the fine clamp, ligated, and transected distally.

Under 25× magnification, the donor heart is transplanted into the anterior neck of the recipient using the end-to-end suture technique for both artery and vein anastomoses. The innominate artery of the donor heart and the right common carotid artery of the recipient are anastamosed first using 11-0 suture, and then the pulmonary artery of the donor heart and the external jugular vein of the recipient are anastomosed using a 11-0 suture. The clamp on the jugular vein is released first after the anastomoses are completed, then the clamp on the carotid artery is removed. The transplanted heart is placed in a suitable position avoiding twisting of the anastomosed vessels before the skin incision is closed.

### 6. Perioperative and postoperative Measurements.

The recipient animal is allowed to wake up and observed carefully after surgery. Arterial and venous blood gas, arterial-venous oxygen differences, serum lactic acid, serum CPK and CPK isoenzymes will all be monitored.
A) Blood sampling during surgery. To study complement activation during hyperacute rejection, 0.2-0.3 ml of blood will be collected from the recipient rats at the following times: 1) arterial blood before the vascular clamp is released (before rejection); 2) graft right ventricle blood about 5 minutes after release of clamp (during rejection), and 3) arterial blood after rejection has completed (about 20 minutes after the clamp is released).
B) Blood sampling after surgery. If the animals survive acute rejection, blood samples will be obtained by tail bleeding of recipient rats every other day for eight days and then every 7 days until the donor heart ceases functioning. The samples will be stored at - 80°C for later tests.
C) Myocardium biopsies. Heart biopsies will be taken at baseline (immediately before graft reperfusion), and at the termination of the study. Each sample will undergo histopathological and immunopathological examination.
D) Cardiac xenograft survival. Survival of the transplanted heart will be determined by daily palpation and ECG monitoring. Rejection is considered complete at the time of heart beat cessation.
E) Complement activation in heart tissue. When the heart is rejected, the rat is killed by injection of sodium pentobarbital and the heart is removed for histopathological or immunohistochemical analysis.

### 7. Histopathological studies.

Once the graft heart beat stops, the rat is killed by overdose of sodium pentobarbital before removing the heart. The hearts will be fixed in 10% formalin solution and sections of the heart will be stained with hematoxylin and eosin. Rejection will be graded as follows: 0 = no interstitial infiltrate; 1 = mild, diffuse interstitial extravasation of RBCs; 2 = increased interstitial edema with a moderate to intense extravasation of RBCs, infiltration of mononuclear cells, and focal myocyte necrosis; 3 = severe inflammatory infiltrate with extensive myocyte necrosis and hemorrhage. Grade represents the most severe histologic damage present. In the long-term survivors, particular attention will be paid to cell infiltration, myocardial fibrosis, and vascular injury.

### 8. Immunopathological measurements of complement activity.

A) Immunofluorescent studies. Samples of biopsy tissues are stained for IgG, IgM, C3, C4, C5b neoantigen, MAC, properdin, fibrinogen, polymorphonuclear neutrophils, and platelets, Affinity isolated, fluorescein isothiocyanate (FITC) goat anti-human antibodies against IgG (γ-chain specific), IgM (µ-chain specific), C4, C3, and C5b will be obtained from Organon-Teknika Corporation (Cappel Research Products, Durham, NC). Properdin and fibrinogen are detected using FITC-conjugated rabbit anti-human antibodies (Atlantic, Stillwater, MN, and Accurate, Westbury, NY). MAC is detected using goat monoclonal antibodies against a neoantigen of MAC. Monocytes and/or granulocytes are detected using murine anti-human CDIIb/CD18 (OKM₁) (Ortho, Raritan, NJ) and platelets are detected using murine anti-human CD9 (BA-2). Murine monoclonal antibodies are detected using affinity-isolated F(ab')₂, FITC-conjugated goat anti-mouse IgG antibodies and rabbit anti-goat IgG antibodies (Organon-Teknika Corporation). Goat monoclonal antibodies and affinity-purified antibodies directed against human immunoglobulin and complement are standardized by reactivity with baboon tissues and by ELISA against baboon serum. Anticomplement reagents are standardized against kidney tissue obtained from a baboon with immune deposits.
B) Preparation and staining of tissue sections. Tissue samples from heart xenografts are snapfrozen in precooled isopentane and liquid nitrogen and stored at -70°C until used. Frozen tissue sections (4 µm thickness) are cut. Tissue sections are air-dried, fixed with acetone, and washed with phosphate-buffered saline (pH 7.4). Tissue sections are then incubated for 45 min with an affinity isolated, FITC-conjugated, labeled primary antibody or with unlabeled monoclonal or polyclonal goat antibodies. Unlabeled goat monoclonal antibodies are detected with a double fluorochrome antibody layer consisting of affinity-isolated, F(ab')₂ FITC-conjugated goat anti-mouse IgG (H and L) and affinity-isolated, F(ab')₂ FITC-conjugated rabbit anti-goat IgG (H and L), both reagents having been absorbed with human serum. After staining, tissues are washed with phosphate-buffered saline and mounted in a solution containing p-phenylenediamine and glycerol. Background immunofluorescence is assessed by omitting the primary antibodies. Antibodies against human immunoglobulin are absorbed with porcine serum. All tissue is examined and photographed using a fluorescence microscope.
C) Measurement of natural antibody levels. Total and xenoreactive IgM titers are measured at baseline and after xenograft. Total IgM is determined by ELISA. Binding of xenoreactive IgM to cultured porcine aortic endothelial cells (anti-PAEC IgM) is determined by ELISA. The anti-Gala (1,3)Gal IgM component of anti-PAEC IgM is determined by measuring the decrease in IgM binding to cultured porcine cells after treatment with a 0.4 U ml⁻¹ solution of αl-3 galactosidase (from the green coffee bean) (Boehringer Mannheim, GMBH, Mannheim, Germany) before ELISA.. Xenoreactive IgM titers are shown for both anti-PAEC IgM and anti-Gal (1,3)Gal IgM.
D) Assays of complement activity. Complement activation is quantified by measurement of baboon plasma C4 functional activity and by total hemolytic (CH50) activity. The plasma samples used for the assays of complement activity are collected in EDTA and stored at -70°C as described above. During processing, all samples are prepared on ice to maintain temperatures below 4°C. Whole complement titration assays are performed using art-known techniques. The plasma levels of the fourth component of complement (C4) are determined using an art-known one-step functional assay.

### 9. Animal groups studied.

Approximately 100 BALB/c mice weighing 20-25 grams will be used as heart donors, and adult Sprague-Dawley rats weighing 200-300 grams will be used as recipients.

The animals will be divided into two groups:
Control group: non-sensitized rats will be used as recipients.
Study group: rats that have been sensitized with donor mouse splenocytes will be used as recipients.

### 10. Results.

It is expected that complement activation will occur in both groups, with rejection after transplantation. The study group, in which the rats have been sensitized, will have a much quicker rejection, referred to as hyperacute rejection. Complement activation in this group is expected to be much more severe than in the control group.

### EXAMPLE 6. The effectiveness of IMP on mouse-to-rat heart xenotransplantation.

This study will test the effectiveness of IMP on complement activation during mouse-to-rat heart xenotransplantation. The study will be divided into three parts: 1) the effectiveness of IMP alone on mouse-to-rat heart transplantation and dose response of IMP; 2) the effect of IMP in combination with lectin: and 3) the effect of IMP in combination with regular immunosuppression.

Mannose binding lectin (MBL) is a C-type lectin involved in the first line of host defense against pathogens. Lectin can block a natural antibody from interacting with tissue surfaces and activating complement. If it is used with IMP, the effect of compliment inhibition may be very much enhanced.

Because of the above combinations, xenografted heart may be able to survive hyperacute rejection and live much longer, We will add triple immunosuppression to further extend its survival time and examine cellular mediated rejection.

### Example 6A. Dose-response of IMP.

### 1. Experimental design

The experiment design is similar to that described in Example 5, above. The recipient rats will be sensitized by mouse splenocyte injection.

### 2. Animal groups studied.

Four groups of heart transplants will be performed. Each group will include 50 transplants.
Group 1: Control group, Mouse-to-rat heart xenotransplantation will be performed, but no IMP will be used.
Group 2: Mouse-to-rat heart xenotransplantation will be performed, and IMP will be given at a dosage of 5 mg/kg/day
Group 3: IMP 15 mg/kg/day
Group 4: IMP 25 mg/kg/day.

### 3. Results.

We expect that IMP will inhibit host complement activation, and xenotransplanted mouse hearts will survive longer than the group without IMP. The most effective dosage of IMP will be chosen.

### Example 6B. The effect of IMP given in combination with lectin.

### 1. Experimental design.

The experiment design is similar to that described in Example 5 (above). The recipient rats will be sensitized by mouse splenocyte injection. Heart samples will be examined for complement deposition as described in Example 2 (above).

### 2. Animal groups studied.

Three groups of heart transplant will be performed. Each group will have 50 transplants.
Group 1: Control group. Mouse-to-rat heart xenotransplantation will be performed, but no IMP will be used.
Group 2: Mouse-to-rat heart xenotransplantation will be performed, and IMP will be used. The best dosage found in Study 4A will be used.
Group 3: Mouse-to-rat heart xenotransplantation plus IMP and lectin (10 mg/kg/day).

### 2. Results

We expect that addition of lectin will further reduce hyperacute rejection and extend heart survival time. Complement activation should also be inhibited more extensively.

### Example 6C. The effect of IMP in combination with regular immunosuppression.

IMP can reduce or eliminate hyperacute rejection. However, the rejection process involved in xenotransplantation is not limited to hyperacute rejection. After the first violent hyperacute rejection, there will be humoral and cellular rejections. These rejections are initiated by the complement system. To increase survival of xenogeneic organ transplant, regular immunosuppression is required. This study is designed to further explore the possibility of extending xenotransplant survival time by combining IMP and regular immunosuppressive treatment.

Because of the addition of immunosuppression, it is possible for the transplanted heart to survive much longer. Within the first three months, a similar acute rejection process, as seen in allograft heart transplantation, will be expected to occur. This rejection is cell-mediated and is an inflammatory infiltrate with various grades of severity. Lymphocyte activation has been a standard approach for evaluating cell-mediated rejection. The CD69 antigen is one of the earliest markers expressed on activated T, B and natural killer (*NK*) lymphocytes following stimulation by antigens. We have used in our laboratory a rapid T-cell activation assay that measures the cell surface expression of CD69 antigen using multiparameter flow cytometry. This allows us to detect and monitor early activation of cell-mediated rejection.

### 1. Experimental design.

A). Heart transplantation surgery will be performed as in Example 5 (above).
B). Dosage of IMP. The dosage of IMP will be chosen according to the most effective dose shown in Example 6A, above.
C) Regular immunosuppression protocol.
   1. Preoperative dosage: cyclosporine A 15 mg/kg, and methylprednisolone 10 mg/kg, iv.
   2. Postoperative dosage: cyclosporine A 15 mg/kg/day, prednisone 0.5 mg/kg/day, and azathioprine 1.5 mg/kg/day, iv.

### 2. Measurement of complement activation.

This measurement will be performed the same way as in Example 4 (above).

### 3. Measurement of lymphocyte activation/CD69 membrane expression.

A) Sample Preparation and Mitogenic Stimuli. Whole blood is collected and placed in containers containing heparin sodium. Blood will be collected from experimental and control animals and placed in heparin containing containers. For controls, various concentrations of control mitogenic lectins (e.g., pokeweed mitogen (PWM), 25 µg/ ml final concentration; Sigma Chemical Company, St. Louis, MO) and antibodies (*e.g*., CD2/CD2R, 10 µg/ml of each; Becton Dickinson Immunocytometry Systems, San Jose, CA) will be added to 500 µl aliquots of whole blood in 12 × 75 mm polystyrene tubes. Other stimuli to be included are the superantigen staphylococcal enterotoxin B (SEB; Sigma) and the specific antigen *Candida albicans* (Greer Laboratories, Inc., Lenoir, NC) at final concentrations of 10 µg/ml and 40 µg/ml, respectively. Optimal concentrations of the stimuli to be used will be determined by titrations using animal donors. Samples will be incubated for 4 hours in a 37°C water bath. A PWM-specific monomeric sugar, N-acetyl-a-D-glucosamine (Aldrich Chemical Company, Inc., Milwaukee, WI), is added to PWM-stimulated samples at 3% final concentration, and 5 mM EDTA is added to all samples postactivation.
B) Three-Color Immunofluorescent Staining. Three-color immunofluorescent staining is performed with modifications to previously published methods. Briefly, 50 µl sample aliquots are stained with 10 µl of a titrated mixture of three monoclonal antibody fluorochrome conjugates for 15 min at room temperature in the dark. For a typical analysis of T-cell activation, the staining antibody combination included CD3 (Leu 4) conjugated with cyanin-5/phycoerythrin (CY-5/PE), CD4 (Leu 3a) or CD8 (Leu 2a) conjugated with fluorescein isothiocyanate (FITC), and CD69 (Leu 23) conjugated with phycoerythrin (PE). IgGI isotope control antibody conjugates are included to establish background fluorescence. All antibodies used in the staining protocols presented in this report are obtained from Becton Dickinson Immunocytometry Systems. All antibody fluorochrome conjugates are prepared with a fluorochrome to protein ratio of 1:1 except for FITC conjugates, which are typically 3 - 4:1. Samples are fixed (unlysed) with 400 µl of 0.5% paraformaldehyde in phosphate-buffered saline (PBS) for 1 hour at 20°C or for 16 hour at 4°C prior to FACS analysis.
C). Flow Cytometric Analysis. Whole blood samples are analyzed by three-color analysis using a FACScan flow cytometer (Becton Dickinson Immunocytometry Systems). Data are acquired with LYSYS 11 software by using fluorescence triggering in the FL3 channel (CY5/PE) to gate on lymphocyte populations (typically, CD3⁺ cells). Data are displayed as two-color dot plots (FLI vs. FL2) to measure the proportion of activated lymphocyte subsets that expressed CD69. Data are analyzed using either LYSYS 11 software on a Hewlett Packard 340 computer or Attractors (Becton Dickinson) cluster analysis software on an Apple Macintosh IICi computer.

### 4. Animal groups studied.

Two groups will be studied, and each group will have 50 animal experiments. The rats in the control group will receive IMP injection only. The dose will be chosen according to Example 6A, above. The rats in the study group will receive IMP, plus regular immunosuppression.

### 5. Expected results.

We expect that after using IMP, the recipient animals will be able to survive hyperacute rejection, and complement activation will be much less severe. With added regular immunosuppression, cell mediated rejection will be less severe. The above combination will make organ survival much longer.

### EXAMPLE 7. Transplantation of hearts from transgenic mice expressing IMP to sensitized rats

### 1. Creation of transgenic mice expressing IMP.

Breeding pairs of transgenic mice either expressing the IMP in its secretory form or expressing IMP as a membrane anchored protein in heart tissue will be generated. Plasmids containing full-length IMP are currently available. However, a new recombinant plasmid will be constructed in which the membrane anchoring region of the membrane glycoprotein B5R of vaccinia virus is spliced at the 3' end of the IMP insert. The cloning and characterization of isogeneic mouse genomic target DNA, as well as the design and generation of the targeting vector, can be achieved with reasonable confidence using art-known techniques. The constructed plasmid will be sequenced and analyzed for correct insertion and expected size membrane bound IMP. Drug selection will be performed, followed by screening for recombinant clones by Southern blot analysis and blastocyst injection of the recombinant clone.

### 2. Transgenic spleen cell and complement activation.

This study will test whether spleen cells from transgenic mice can be protected against human complement. The study will be performed the same way as in Example 5 (above). However, splenocytes from transgenic mice will be used instead of splenocytes from regular mice. The measurement of C3 deposition will be the same as in Example 5 (above).

### 3. Heart transplantation from transgenic mice to sensitized rats.

The procedure for cervical heart transplantation will be the same as in Example 6 (above). The donor heart will be obtained from transgenic mice expressing IMP.

### 4. Postoperative measurements.

Postoperative observations of heart function will be the same as in Example 6 (above). Laboratory tests for complement activation will also be the same as in Example 6.

### 5. Animal groups studied.

During transgenic mouse heart transplantation, two animal groups will be used. There will be 50 mouse-to-rat transplants in each group. For rats in the control group, regular mouse will be used as heart donor. For rats in study group 1, transgenic mouse heart will be used for transplantation. No extra immunosuppression will be used. For rats in study group 2, transgenic mouse heart will be used for transplantation, and regular immunosuppression (same as Example 6C) will be used postoperatively.

### 6. Results.

We expect that complement activation will be much less severe in the rats receiving hearts from transgenic mice. The combined use of immunosuppression will further increase survival of the recipient mice.

### EXAMPLE 8. Ability of IMP to block attachment of antibody to tissue surfaces

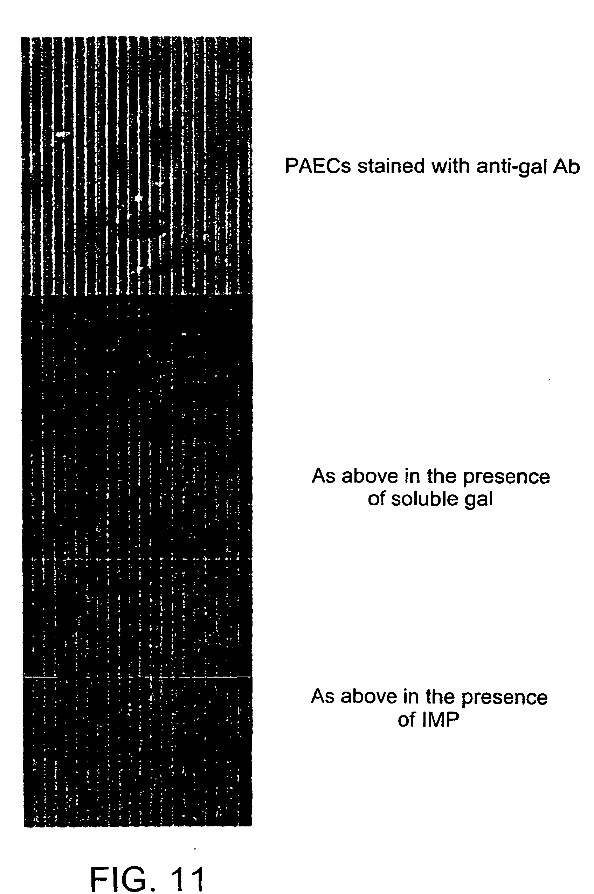

The present inventors have discovered that IMP is able to block natural human antibody or anti-gal antibody from binding to pig endothelial cells (Fig. 10 and 11). Figure 10 illustrates the inhibition of binding of anti-alpha-gal rhodamine conjugated antibody to pig aortal endothelial cells (PAECs) in the presence of alpha gal or IMP. The top panel shows red immunofluorescence due to the alpha gal antibody binding to the PAECs. The middle panel shows that alpha gal competes for binding to the antibody and the intensity of fluorescence is significantly diminished. The bottom panel also shows significant reduction in fluorescence due to the IMP binding to the heparin on the cell surface of PAECs and stearically hindering the binding of the rhodamine-conjugated anti-alpha gal antibody.

Figure 11 displays the results of flow cytometry showing inhibition of binding (decrease in fluorescence intensity) of anti-alpha gal antibody in the presence of IMP as evidenced by a shift in the peak toward lower intensity. This confirms the result shown in Fig. 10, and the conclusion is the same.

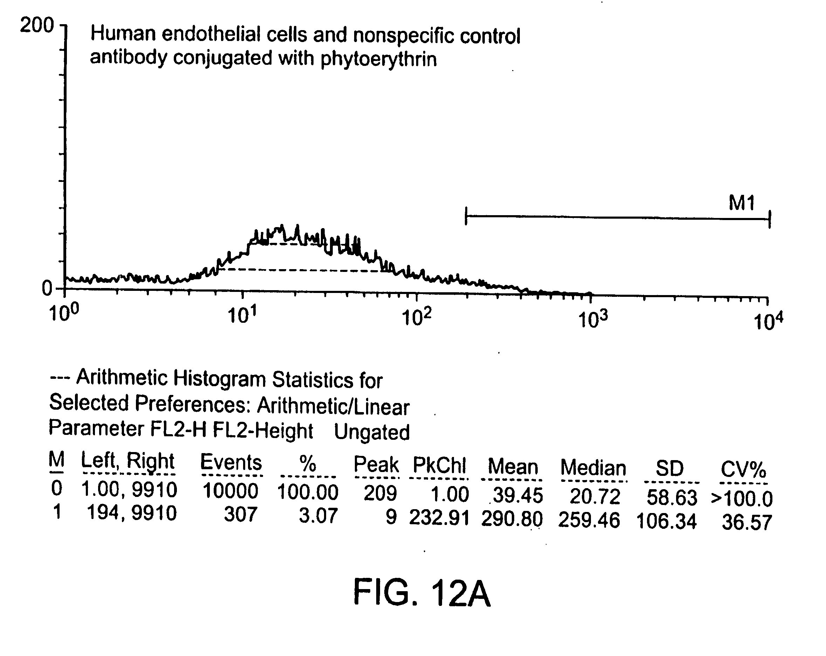

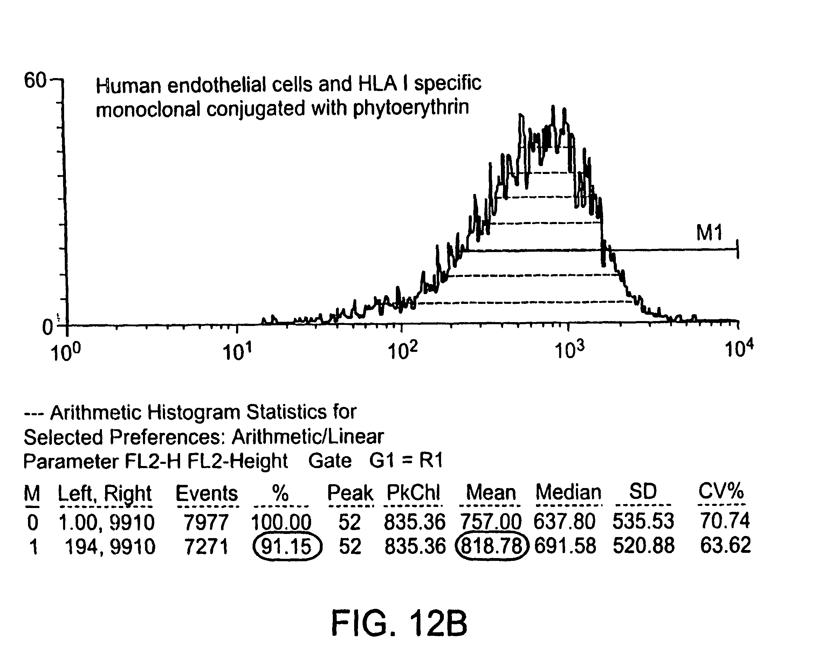

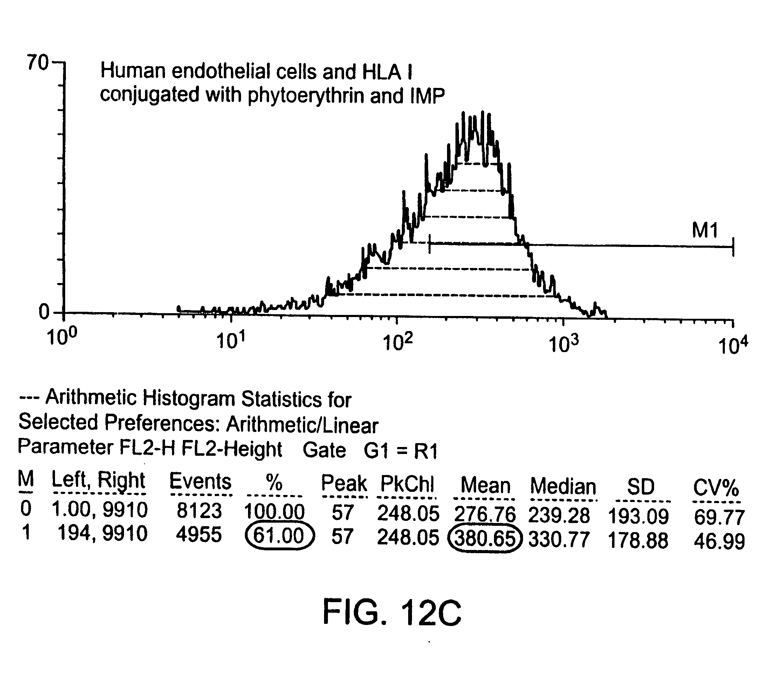

The present inventors have also discovered that IMP is able to block binding of mouse anti-HLA antibody to human umbilical vascular endothelial cells (HuVECs) (Fig. 12). The results of an evaluation of whether the binding of antibody to PAECs is specific only to the alpha gal residues on the surface or that the stearic hindrance does not discriminate specific from non-specific antibodies are shown in Fig. 12. Human endothelial cells were treated with either control antibody treatment (panel A), anti-human leukocyte antigen antibody conjugated with phycoerythrin (Panel B), or IMP along with the PAECs and the anti-HLA I antibody (Panel C). The results of this experiment show that the fluorescence after correction for non-specific fluorescence (control antibody) was reduced to 25% in the presence of IMP. These data lead to the conclusion that IMP nonspecifically blocks any antibody binding to the endothelial cells.

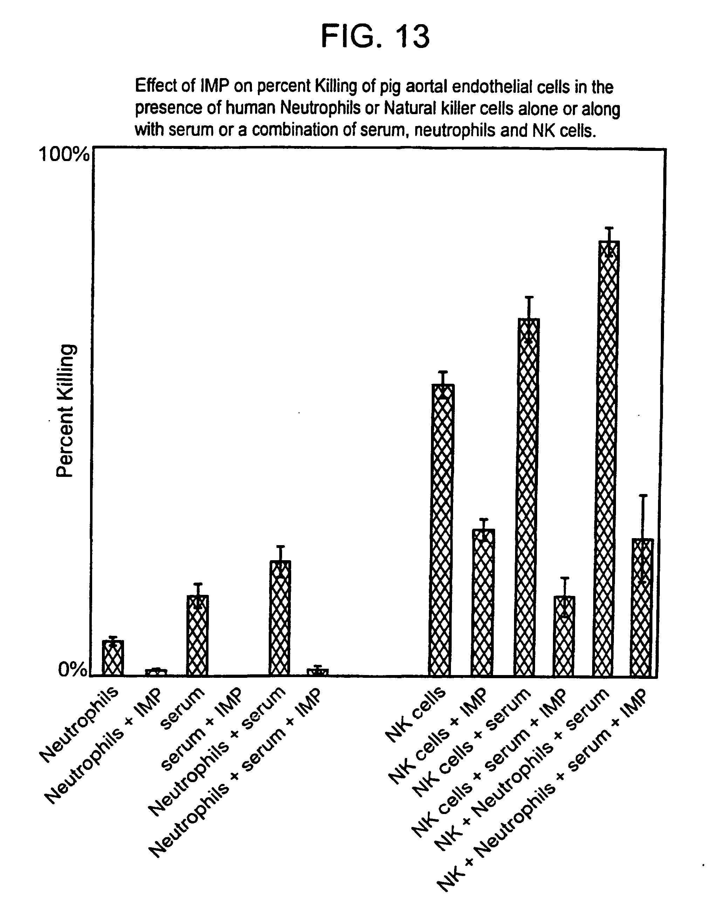

The present inventors have also found that IMP is able to block killing of pig aortal endothelial cells by human neutrophils and natural killer cells alone or in the presence of human serum or NK cells, neutrophils, and serum all at the same time (Fig. 13). There was consistently lesser killing of any of the combinations in the presence of IMP in comparison to without IMP. These data lead to the conclusion that IMP blocks complement mediated killing as well as the killing by cytotoxic cells. The latter inhibition is probably due to the IMP binding to heparin blocking the cell-cell interactions.

While the invention has been described and illustrated herein by references to various specific material, procedures and examples, it is understood that the invention is not restricted to the particular material, combinations of material, and procedures selected for that purpose. Numerous variations of such details can be implied and will be appreciated by those skilled in the art.

## Claims

1. Use of a protein comprising the amino acid sequence of residues 20 to 263 of SEQ ID NO:1, or a pharmaceutically acceptable salt thereof, for the manufacture of a medicament for preventing or treating xenograft rejection in a patient in need of such treatment.

2. The use according to claim 1 wherein the amino acid sequence of said protein consists of the amino acid sequence of SEQ ID NO:1.

3. The use according to any of claims 1 to 2, wherein an immunosuppressive agent is coadministered to the patient.

4. The use according to claim 3, in which the medicament further comprises the immunosuppressive agent.

5. The use according to claim 3 or 4, wherein the immunosuppressive agent is selected from the group consisting of azathioprene, cyclosporine, and pharmaceutically acceptable salts thereof.

6. The use according to any one of claims 3 to 5, wherein a glucocorticoid is coadministered to the patient.

7. The use according to claim 6, in which the medicament further comprises the glucocorticoid.

8. The use according to claim 6 or 7, wherein the glucocorticoid is prednisone.

9. The use according to any one of claims 1 to 8 wherein the medicament additionally comprises a pharmaceutically acceptable carrier.

10. The use according to claim 9, wherein the carrier is aqueous.

## Patentansprüche

1. Verwendung eines Proteins, umfassend die Aminosäuresequenz der Reste 20 bis 263 von SEQ ID NO:1, oder ein pharmazeutisch annehmbares Salz davon, zur Herstellung eines Medikaments zum Verhindern oder Behandeln einer Xenotransplantatabstoßung bei einem Patienten, der einer solchen Behandlung bedarf.

2. Verwendung nach Anspruch 1, wobei die Aminosäuresequenz des Proteins aus der Aminosäuresequenz von SEQ ID NO:1 besteht.

3. Verwendung nach einem der Ansprüche 1 bis 2, wobei dem Patienten gleichzeitig ein immunsuppressiver Wirkstoff verabreicht wird.

4. Verwendung nach Anspruch 3, wobei das Medikament weiter den immunsuppressiven Wirkstoff umfasst.

5. Verwendung nach Anspruch 3 oder 4, wobei der immunsuppressive Wirkstoff aus der Gruppe bestehend aus Azathiopren, Cyclosporin und pharmazeutisch annehmbare Salze davon ausgewählt ist.

6. Verwendung nach einem der Ansprüche 3 bis 5, wobei dem Patienten gleichzeitig ein Glucocorticoid verabreicht wird.

7. Verwendung nach Anspruch 6, wobei das Medikament weiter das Glucocorticoid umfasst.

8. Verwendung nach Anspruch 6 oder 7, wobei das Glucocorticoid Prednison ist.

9. Verwendung nach einem der Ansprüche 1 bis 8, wobei das Medikament zusätzlich einen pharmazeutisch annehmbaren Träger umfasst.

10. Verwendung nach Anspruch 9, wobei der Träger wässrig ist.

## Revendications

1. Utilisation d'une protéine comprenant la séquence d'acides aminés de résidus 20 à 263 de SEQ ID No : 1, où un sel acceptable d'un point de vue pharmaceutique pour fabriquer un médicament destiné à empêcher ou traiter le rejet lors de la xénogreffe chez un patient qui a besoin d'un traitement de ce type.

2. Utilisation selon la revendication 1 dans laquelle la séquence d'acides aminés de ladite protéine est constituée de la séquence d'acides aminés de SEQ ID No: 1.

3. Utilisation selon une des revendications 1 à 2, où un immunodépresseur est co-administré au patient.

4. Utilisation selon la revendication 3, dans laquelle le médicament comprend, en outre, l'immunodépresseur.

5. Utilisation selon la revendication 3 ou 4, où l'immunodépresseur est choisi parmi le groupe comprenant de l'azathioprene, de la cyclosporine et des sels de ce groupe acceptables d'un point de vue pharmaceutique

6. Utilisation selon n'importe laquelle des revendications 3 à 5, où un glucocorticoïde est co-administré au patient.

7. Utilisation selon la revendication 6, où le médicament comprend, en outre, le glucocorticoïde.

8. Utilisation selon la revendication 6 ou 8, où le glucocorticoïde est de la prednisone.

9. Utilisation selon n'importe laquelle des revendications 1 à 8, où le médicament comprend, en outre, un porteur acceptable d'un point de vue pharmaceutique.

10. Utilisation selon la revendication 9, où le porteur est aqueux.
